# EUROPEAN PATENT SPECIFICATION

(11) **EP 2 536 500 B1**
(45) Date of publication and mention of the grant of the patent: **07.12.2016**
(21) Application number: 11704850.4
(22) Date of filing: 17.02.2011
(51) Int. Cl.: B01J 31/02

(54) **SUPPORTED METAL CATALYSTS**
GETRÄGERTE METALLKATALYSATOREN
CATALYSEURS METALLIQUES SUPPORTES

(30) Priority: 17.02.2010 GB 201002677
(43) Date of publication of application: 26.12.2012
(73) Proprietor: Johnson Matthey PLC, London EC4A 4AB (GB)
(72) Inventor: BISHOP, Peter Trenton, Oxfordshire RG4 9LS (GB); COOKSON, James, Oxfordshire OX11 9JS (GB); HANCOCK, Frederick Earnest, Hertfordshire SG8 6AP (GB); HAWKER, Steven, Hertfordshire SG8 7DF (GB); MCNAIR, Robert John III, Mullica Hill New Jersey 08062 (US)
(74) Representative: Whitcombe, Nicole Jane
(86) International application number: PCT/GB2011/050321
(87) International publication number: WO 2011/101679

(56) References cited:
- CN-A- 101 549 296
- MHADGUT, SHILPA C. ET AL: "Nature of Proline -induced Enantiodifferentiation in Asymmetric Pd Catalyzed Hydrogenations: Is the Catalyst Really Indifferent?", 2008, CATALYSIS LETTERS , 123(1-2), 156-163 CODEN: CALEER; ISSN: 1011-372X, XP002630898, table 2
- MONDELLI, CECILIA ET AL: "Fundamental Aspects of the Chiral Modification of Platinum with Peptides: Asymmetric Induction in Hydrogenation of Activated Ketones", 2009, JOURNAL OF PHYSICAL CHEMISTRY C , 113(34), 15246-15259 CODEN: JPCCCK; ISSN: 1932-7447, XP002630899, * abstract
- GOBOLOS, S. ET AL: "Asymmetric synthesis of (S)-alkylamines via reductive transamination of ketones over carbon - supported palladium catalysts", 1999, JOURNAL OF MOLECULAR CATALYSIS A: CHEMICAL , 146(1-2), 129-138 CODEN: JMCCF2; ISSN: 1381-1169, XP002630900, page 130, column 1, paragraph 2 page 130, column 2, paragraph 2
- SZOLLOSI, G. ET AL: "Heterogeneous asymmetric reactions. Part 21. Amino acid derived modifiers in the enantioselective hydrogenation of ethyl pyruvate over supported platinum catalyst", 2001, JOURNAL OF MOLECULAR CATALYSIS A: CHEMICAL , 170(1-2), 165-173 CODEN: JMCCF2; ISSN: 1381-1169, XP002630901, * abstract; figure 1
- DATABASE CA [Online] CHEMICAL ABSTRACTS SERVICE, COLUMBUS, OHIO, US; TAN, LIN ET AL: "Direct Determination of Uric Acid Based on Pd Nanoparticles Electrodepositing onto Anatase-Type TiO2 Nanoparticles/Chitosan Film-Modified Electrode", XP002630902, retrieved from STN Database accession no. 2008:1453387
- MA S ET AL: "Direct synthesis of hydrogen peroxide from H2/O2 and oxidation of thiophene over supported gold catalysts", CHEMICAL ENGINEERING JOURNAL, ELSEVIER SEQUOIA, LAUSANNE, CH, vol. 156, no. 3, 1 February 2010 (2010-02-01), pages 532-539, XP026855436, ISSN: 1385-8947 [retrieved on 2009-04-10]
- KOUACHI K ET AL: "Preparation of silica-supported cobalt catalysts from water-in-oil microemulsion for selective hydrogenation of citral", JOURNAL OF MOLECULAR CATALYSIS A: CHEMICAL, ELSEVIER, AMSTERDAM, NL, vol. 308, no. 1-2, 4 August 2009 (2009-08-04), pages 142-149, XP026266457, ISSN: 1381-1169, DOI: DOI:10.1016/J.MOLCATA.2009.04.001 [retrieved on 2009-04-09]
- COVERT, LLOYD W. ET AL: "Use of nickel as a catalyst for hydrogenation. II", 1932, JOURNAL OF THE AMERICAN CHEMICAL SOCIETY , 54, 1651-63 CODEN: JACSAT; ISSN: 0002-7863, XP002630903, table 3
- MASTALIR A ET AL: "Synthesis and catalytic application of Pd nanoparticles in graphite oxide", CARBON, ELSEVIER, OXFORD, GB, vol. 46, no. 13, 1 November 2008 (2008-11-01), pages 1631-1637, XP025469425, ISSN: 0008-6223, DOI: DOI:10.1016/J.CARBON.2008.06.054 [retrieved on 2008-07-08]
- L'ARGENTIERE P C ET AL: "A palladium tetra-coordinated complex as catalyst in the selective hydrogenation of 1-heptyne", APPLIED CATALYSIS A: GENERAL, ELSEVIER SCIENCE, AMSTERDAM, NL, vol. 226, no. 1-2, 28 March 2002 (2002-03-28), pages 253-263, XP004335978, ISSN: 0926-860X, DOI: DOI:10.1016/S0926-860X(01)00911-5
- DRELINKIEWICZ A ET AL: "Amine groups functionalized gel-type resin supported Pd catalysts: Physicochemical and catalytic properties in hydrogenation of alkynes", JOURNAL OF MOLECULAR CATALYSIS A: CHEMICAL, ELSEVIER, AMSTERDAM, NL, vol. 300, no. 1-2, 2 March 2009 (2009-03-02), pages 8-18, XP026626169, ISSN: 1381-1169, DOI: DOI:10.1016/J.MOLCATA.2008.10.035 [retrieved on 2008-10-31]
- CARDENAS-LIZANA F ET AL: "Pd-promoted selective gas phase hydrogenation of p-chloronitrobenzene over alumina supported Au", JOURNAL OF CATALYSIS, ACADEMIC PRESS, DULUTH, MN, US, vol. 262, no. 2, 10 March 2009 (2009-03-10), pages 235-243, XP025988837, ISSN: 0021-9517, DOI: DOI:10.1016/J.JCAT.2008.12.019 [retrieved on 2009-01-22]
- MALLAT T ET AL: "Selectivity enhancement in heterogeneous catalysis induced by reaction modifiers", APPLIED CATALYSIS A: GENERAL, ELSEVIER SCIENCE, AMSTERDAM, NL, vol. 200, no. 1-2, 28 August 2000 (2000-08-28), pages 3-22, XP004272444, ISSN: 0926-860X, DOI: DOI:10.1016/S0926-860X(00)00645-1

## Description

The present invention relates to supported metal catalysts, wherein the catalysts are modified by at least one amine, a method for the preparation thereof and hydrogenation processes utilising the supported metal catalysts.

WO2008098830 (to Evonik Degussa GmbH) relates to supported and unsupported transition metal catalysts which surfaces have been modified with sulfur-containing modifiers. Example 2 describes the preparation of modified Pt catalysts wherein H₂PtCl₆.6H₂O is reduced onto Al₂O₃. The Pt/Al₂O₃ catalyst is then suspended in methanol and modified by the addition of a modifier.

Ma et al (Chemical Engineering Journal 156 (2010) 532-539) relates to the direct synthesis of hydrogen peroxide from H₂/O₂ and oxidation of thiophene over supported gold catalysts. Mastalir et al (Carbon 46 (2008) 1631-1637) relates to the synthesis and catalytic application of Pd nanoparticles in graphite oxide. L'Argentière et al (Applied Catalysis A: General 226 (2002) 253-263) relates to a palladium tetra-coordinated complex as catalyst in the selective hydrogenation of 1-heptyne. Drelinkiewicz et al (Journal of Molecular Catalysis A: Chemical 300 (2009) 8-18) relates to amine groups functionalized gel-type resin supported Pd catalysts. Cardenas-Lizana et al (Journal of Catalysis 262 (2009) 235-243) relates to Pd-promoted selected gas phase hydrogenation of *p*-chloronitrobenzene over alumina supported Au.

### Summary of the Invention

In one aspect the present invention provides a supported metal catalyst, wherein the catalyst comprises at least one metal selected from Group VIII or IB of the Periodic Table, the catalyst is modified by at least one amine, and wherein the catalyst comprises faceted particles,
provided that when the metal is Pt and the support is Al₂O₃, the amine is not:
a) S-benzyl-L-cysteine;
b) N-benzyl-S-benzyl-L-cysteine;
c) L-cysteine ethyl ester;
d) S-benzyl-L-cysteine ethyl ester;
e) N-benzyl-S-benzyl-L-cysteine ethyl ester;
f) S-phenyl-L-cysteine ethyl ester; or
g) N-benzyl-S-phenyl-L-cysteine ethyl ester.

Another aspect of the invention provides a process for the preparation of a supported metal catalyst as defined herein, wherein the process comprises the steps of:
a) mixing a support, at least one water soluble metal salt and at least one amine in an aqueous solvent; and
b) adding a reducing agent to form the supported metal catalyst;
and wherein steps (a) and (b) are carried out as a one-pot reaction.

Yet another aspect of the invention provides a process for the preparation of an optionally substituted amine, comprising the step of hydrogenating an optionally substituted benzyl-amine in the presence of hydrogen and a supported metal catalyst as described herein.

Another aspect of the invention provides a process for the preparation of an optionally substituted arylamine, comprising the step of hydrogenating an optionally substituted aryl compound comprising one or more nitro groups in the presence of hydrogen and a supported metal catalyst as defined herein.

Yet another aspect of the invention provides a process for the preparation of an optionally substituted alkene, comprising the step of hydrogenating an optionally substituted alkyne in the presence of hydrogen and a supported metal catalyst as defined herein.

### Definitions

The point of attachment of a moiety or substituent is represented by "-". For example, -OH is attached through the oxygen atom.

"Alkyl" refers to a straight-chain, branched or cyclic saturated hydrocarbon group. In certain embodiments, the alkyl group may have from 1-20 carbon atoms, in certain embodiments from 1-15 carbon atoms, in certain embodiments, 1-8 carbon atoms. The alkyl group may be unsubstituted or substituted. Unless otherwise specified, the alkyl group may be attached at any suitable carbon atom and, if substituted, may be substituted at any suitable atom. Typical alkyl groups include but are not limited to methyl, ethyl, n-propyl, iso-propyl, cyclopropyl, n-butyl, iso-butyl, sec-butyl, tert-butyl, cyclobutyl, n-pentyl, cyclopentyl, n-hexyl, cyclohexyl and the like.

"Alkenyl" refers to a straight-chain, branched or cyclic unsaturated hydrocarbon group having at least one carbon-carbon double bond. The group may be in either the cis- or trans-configuration around each double bond. In certain embodiments, the alkenyl group can have from 2-20 carbon atoms, in certain embodiments from 2-15 carbon atoms, in certain embodiments, 2-8 carbon atoms. The alkenyl group may be unsubstituted or substituted. Unless otherwise specified, the alkenyl group may be attached at any suitable carbon atom and, if substituted, may be substituted at any suitable atom. Examples of alkenyl groups include but are not limited to ethenyl (vinyl), 2-propenyl (allyl), 1-methylethenyl, 2-butenyl, 3-butenyl, cyclobut-1,3-dienyl and the like.

"Alkynyl" refers to a straight-chain, branched or cyclic unsaturated hydrocarbon group having at least one carbon-carbon triple bond. In certain embodiments, the alkynyl group can have from 2-20 carbon atoms, in certain embodiments from 2-15 carbon atoms, in certain embodiments, 2-8 carbon atoms. The alkynyl group may be unsubstituted or substituted. Unless otherwise specified, the alkynyl group may be attached at any suitable carbon atom and, if substituted, may be substituted at any suitable atom. Examples of alkynyl groups include but are not limited to ethynyl, prop-1-ynyl, prop-2-ynyl, 1-methylprop-2-ynyl, but-1-ynyl, but-2-ynyl, but-3-ynyl and the like.

"Aryl" refers to an aromatic carbocyclic group. The aryl group may have a single ring or multiple condensed rings. In certain embodiments, the aryl group can have from 6-20 carbon atoms, in certain embodiments from 6-15 carbon atoms, in certain embodiments, 6-12 carbon atoms. The aryl group may be unsubstituted or substituted. Unless otherwise specified, the aryl group may be attached at any suitable carbon atom and, if substituted, may be substituted at any suitable atom. Examples of aryl groups include, but are not limited to, phenyl, naphthyl, anthracenyl and the like.

"Arylalkyl" refers to an optionally substituted group of the formula aryl-alkyl-, where aryl and alkyl are as defined above.

"Halo" refers to -F, -Cl, -Br and -I.

"Heteroalkyl" refers to a straight-chain or branched saturated hydrocarbon group wherein one or more carbon atoms are independently replaced with one or more heteroatoms (e.g. nitrogen, oxygen, phosphorus and/or sulfur atoms). The heteroalkyl group may be unsubstituted or substituted. Unless otherwise specified, the heteroalkyl group may be attached at any suitable atom and, if substituted, may be substituted at any suitable atom.

"Heterocycloalkyl" refers to a saturated cyclic hydrocarbon group wherein one or more carbon atoms are independently replaced with one or more heteroatoms (e.g. nitrogen, oxygen, phosphorus and/or sulfur atoms). The heterocycloalkyl group may be unsubstituted or substituted. Unless otherwise specified, the heterocycloalkyl group may be attached at any suitable atom and, if substituted, may be substituted at any suitable atom. Examples of heterocycloalkyl group include but are not limited to epoxide, morpholinyl, piperadinyl, piperazinyl, thirranyl and the like.

"Heteroaryl" refers to an aromatic carbocyclic group wherein one or more carbon atoms are independently replaced with one or more heteroatoms (e.g. nitrogen, oxygen, phosphorus and/or sulfur atoms). Unless otherwise specified, the heteroaryl group may be attached at any suitable atom and, if substituted, may be substituted at any suitable atom. Examples of heteroaryl groups include but are not limited to furanyl, indolyl, oxazolyl, pyridinyl, pyrimidinyl, thiazolyl, thiphenyl and the like.

"Substituted" refers to a group in which one or more (e.g. 1, 2, 3, 4 or 5) hydrogen atoms are each independently replaced with substituents which may be the same or different. Examples of substituents include but are not limited to -halo, -C(halo)₃, -R^{a}, =O, =S, -O-R^{a}, -S-R^{a},-NR^{a}R^{b}, =NR^{a}, =N-OR^{a}, -CN, -SCN, -NCS, -NO₂, -C(O)-R^{a}, -COOR^{a}, -C(S)-R^{a}, -C(S)OR^{a},-S(O)₂OH, -S(O)₂-R^{a}, -S(O)₂NR^{a}R^{b}, -O-S(O)-R^{a} and -CON^{a}N^{b}; wherein R^{a} and R^{b} are independently selected from the groups consisting of H, alkyl, aryl, arylalkyl, heteroalkyl, heteroaryl, heteroaryl-alkyl-, or R^{a} and R^{b} together with the atom to which they are attached form a heterocycloalkyl group, and wherein R^{a} and R^{b} may be unsubstituted or further substituted as defined herein.

### Detailed description

### Supported Metal Catalysts

In one aspect, the present invention provides a supported metal catalyst, wherein the catalyst comprises at least one metal selected from Group VIII or IB of the Periodic Table, the catalyst is modified by at least one amine, and wherein the catalyst comprises faceted particles, provided that when the metal is Pt and the support is Al₂O₃, the amine is not:
a) S-benzyl-L-cysteine;
b) N-benzyl-S-benzyl-L-cysteine;
c) L-cysteine ethyl ester;
d) S-benzyl-L-cysteine ethyl ester;
e) N-benzyl-S-benzyl-L-cysteine ethyl ester;
f) S-phenyl-L-cysteine ethyl ester; or
g) N-benzyl-S-phenyl-L-cysteine ethyl ester.

A supported metal catalyst typically comprises an inert support material and a catalytically active material. The support may be selected from the group consisting of carbon, alumina, calcium carbonate, titania, silica, zirconia, ceria and a combination thereof. When the support is alumina, the alumina may be in the form of alpha-Al₂O₃, beta-Al₂O₃, gamma-Al₂O₃, delta-Al₂O₃, theta-Al₂O₃ or a combination thereof. When the support is carbon, the carbon may be in the form of activated carbon (e.g. neutral, basic or acidic activated carbon), carbon black or graphite (e.g. natural or synthetic graphite). Examples of suitable carbon supports are Norit Carbon GSX, Ceca L4S, Ceca 2S, Ceca CPL, Timcal T44 Graphite or a combination thereof.

The catalyst comprises at least one metal is selected from Group VIII or IB of the Periodic Table. More preferably, the metal is selected from the group consisting of at least one of the platinum group metals (i.e. Pd, Pt, Ru, Rh, Ir and Os), the coinage metals (i.e. Cu, Ag and Au), iron, cobalt and nickel. Most preferably, the metal is palladium, platinum and/or gold.

In one embodiment, the supported metal catalyst comprises a single metal e.g. Pt, Pd or Au.

In another embodiment, the supported metal catalyst comprises two metals e.g. Au-Pd, Au-Pt or Pd-Au. The ratio of each metal may be any suitable ratio. In one embodiment, the ratio is from about 0.01: 1 wt% to about 20:1 wt% with respect to each metal, more preferably from about 0.1:1 to about 10:1 wt% e.g. 0.5:1, 1:1, 2:1, 4:1 or 9:1 wt%.

Whether the supported metal catalysts comprises a single metal or two or more metals, the metal loading of the supported metal catalyst may be any suitable loading, such as from about 0.01 wt% to about 20 wt% per metal.

The at least one amine is preferably selected from the group consisting of natural amino acids, non-natural amino acids, peptides, substituted or unsubstituted alkylamines, substituted or unsubstituted alkyldiamines, substituted or unsubstituted alkylpolyamines and combinations thereof. In one embodiment, the substituted or unsubstituted alkylamine, alkyldiamine or alkylpolyamines have from 1-20 carbon atoms and in certain embodiments, 1-15 carbon atoms.

Natural amino acids and their nomenclature are well-known in the art, for example, see Biochem. J., 1984, 219, 345. By "non-natural amino acids" we mean a compound comprising an amino and a carboxylic acid group but which is not a natural amino acid. Examples, of non-natural amino acids are hydroxylysine, hydroxyproline, alloleucine, allotheonine, aminovaleric acid, aminohexanoic acid, homoserine, homoarginine, homophenylalanine, aminopropanoic acid, aminopropanoic acid, aminobutyric acid, aminopentanoic acid, aminohexanoic acid, aminohexandioic acid, aminoheptandioic acid, diaminopropanoic acid, diaminobutanoic acid, diaminopentanoic acid, diaminoheptandioic acid, carboxyglutamic acid, butylglycine, chlorophenylalanine dichlorophenylalanine, cyclohexylalanine, citrulline, dehydrophenylalanine, fluorophenylalanine, indolecarboxylic acid, iodophenylalanine, naphthylalanine, phenylglycine, O-acetylphenylserine, pyridylalanine, sarcosine, 1,2,3,4-tetrahydroisoquinoline-3-carboxylic acid, O-methyltyrosine, caproic acid and isomers thereof.

The amino acid (whether natural or non-natural) may have the D-, L- or DL- configuration.

In one embodiment, it is preferred that the natural amino acid or non-natural amino acid does not comprise a sulfur-containing functionality as the sulfur-containing functionality may poison the catalyst.

When the at least one amine is a peptide, it is preferred that the peptide consists of 2, 3, 4, 5 or more amino acids. The amino acids may be natural and/or non-natural as described above. Examples of peptides are GlyGly and GlyGlyGly.

Preferably, the at least one amine is selected from the group consisting of lysine, glycine, proline, alanine, serine, phenylalanine, asparagine, aspartic acid, valine, butylamine, 6-aminocaproic acid, 1,6-diaminohexane, hexylamine and combinations thereof.

The ratio of amine : metal may be any suitable ratio. In one preferred embodiment, however, the ratio is from about 0.05:1 to about 5:1.

Preferably, the supported metal catalyst is selected from the group consisting of:

| **Metal(s)** | **Support** | **Amine** |
|---|---|---|
| Palladium | Carbon | Lysine |
| Palladium | Carbon | Glycine |
| Palladium | Carbon | Proline |
| Palladium | Carbon | Alanine |
| Palladium | Carbon | Arginine |
| Palladium | Carbon | Serine |
| Palladium | Carbon | Phenylalanine |
| Palladium | Carbon | Asparagine |
| Palladium | Carbon | Aspartic acid |
| Palladium | Carbon | Valine |
| Palladium | Carbon | Butylamine |
| Palladium | Carbon | 6-Amino caproic acid |
| Palladium | Carbon | 1,6-Diaminohexane |
| Palladium | Carbon | Hexylamine |
| Palladium | Alumina | Glycine |
| Palladium | Carbon | Gly-Gly |
| Palladium | Carbon | Gly-Gly-Gly |
| Platinum | Carbon | Lysine |
| Gold | Carbon | Lysine |
| Gold-palladium | Carbon | Lysine |
| Gold-platinum | Carbon | Lysine |
| Palladium-platinum | Carbon | Lysine |

The catalyst may comprise crystallites. In this instance, the crystallites may vary in size from about 1nm to about 50 nm. In one embodiment, the crystallites may be ≥ about 4nm. In another embodiment, the crystallites may be ≤ about 40nm.

The catalyst comprises faceted particles. Without wishing to be bound by theory, it is believed that the large planes and lack of corner sites in the faceted particles may contribute to the selective nature of the supported metal catalysts in hydrogenation reactions. In certain embodiments, the faceted particles may display clear grain boundaries. Without wishing to be bound by theory, this may indicate that the particles grew together from two different nucleation points.

The form of the metal in the supported catalyst may comprise the elemental metal and/or a metal oxide and/or metal hydride. For clarity, however, the elemental metal, metal oxide and/or metal hydride will be referred to by the metal name. For example, a supported palladium catalyst may comprise elemental (metallic) palladium, palladium oxide and/or metal hydride. Regardless of the actual form(s) of palladium present, however, the catalyst will be referred to as a "supported palladium catalyst".

In another aspect, the present invention provides a process for the preparation of a supported metal catalyst as described above, wherein the process comprises the steps of:
(a) mixing a support, at least one water soluble metal salt and at least one amine in an aqueous solvent; and
(b) adding a reducing agent to form the supported metal catalyst;
and wherein steps (a) and (b) are carried out as a one-pot reaction.

The at least one water soluble metal salt may be selected from the group consisting of:
(i) M₂PtX₄ wherein M is H, Li, Na, K or NH₃ and X is Cl, Br, I, NO₃, OH or CN.
   Examples include but are not limited to H₂PtCl₄, Na₂PtCl₄, K₂PtCl₄, Li₂PtCl₄, (NH₃)₂PtCl₄, H₂PtBr₄, Na₂PtBr₄, K₂PtBr₄, Li₂PtBr₄, (NH₃)₂PtBr₄, H₂Ptl₄, Na₂Ptl₄, K₂Ptl₄, Li₂Ptl₄, (NH₃)₂Ptl₄, H₂Pt(NO₃)₄, Na₂Pt(NO₃)₄, K₂Pt(NO₃)₄, Li₂Pt(NO₃)₄, (NH₃)₂Pt(NO₃)₄, H₂Pt(OH)₄, Na₂Pt(OH)₄, K₂Pt(OH)₄, Li₂Pt(OH)₄, (NH₃)₂Pt(OH)₄, H₂Pt(CN)₄, Na₂Pt(CN)₄, K₂Pt(CN)₄, Li₂Pt(CN)₄, (NH₃)₂Pt(CN)₄;
(ii) M₂PtX₆ wherein M is H, Li, Na, K or NH₃ and X is Cl, Br, I, NO₃, OH or CN.
   Examples include but are not limited to H₂PtCl₆, Na₂PtCl₆, K₂PtCl₆, Li₂PtCl₆, (NH₃)₂PtCl₆, H₂PtBr₆, Na₂PtBr₆, K₂PtBr₆, Li₂PtBr₆, (NH₃)₂PtBr₆, H₂Ptl₆, Na₂Ptl₆, K₂Ptl₆, Li₂Ptl₆, (NH₃)₂Ptl₆, H₂Pt(NO₃)₆, Na₂Pt(NO₃)₆, K₂Pt(NO₃)₆, Li₂Pt(NO₃)₆, (NH₃)₂Pt(NO₃)₆, H₂Pt(OH)₆, Na₂Pt(OH)₆, K₂Pt(OH)₆, Li₂Pt(OH)₆, (NH₃)₂Pt(OH)₆, H₂Pt(CN)₆, Na₂Pt(CN)₆, K₂Pt(CN)₆, Li₂Pt(CN)₆, (NH₃)₂Pt(CN)₆;
(iii) PtX₂ wherein X is Cl, Br, I, NO₃, OH or CN.
   Examples include but are not limited to PtCl₂, PtBr₂, Ptl₂, Pt(NO₃)₂, Pt(OH)₂, Pt(CN)₂;
(iv) PtX₄ wherein X is Cl, Br, I, NO₃, OH or CN.
   Examples include but are not limited to PtCl₄, PtBr₄, Ptl₄, Pt(NO₃)₄, Pt(OH)₄, Pt(CN)₄;
(v) Pt(NH₃)_{4-y}X_{y} wherein X is Cl, Br, I or NO₃ and y is 0, 1, 2, 3 or 4.
   Examples include but are not limited to Pt(NH₃)₂Cl₂, Pt(NH₃)₂Br₂, Pt(NH₃)₂l₂, Pt(NH₃)₂(NO₃)₂;
(vi) M₂PdX₄ wherein M is H, Li, Na, K or NH₃ and X is Cl, Br, I, NO₃, OH, CN or HCO₃. Examples include but are not limited to H₂PdCl₄, Na₂PdCl₄, K₂PdCl₄, Li₂PdCl₄, (NH₃)₂PdCl₄, H₂PdBr₄, Na₂PdBr₄, K₂PdBr₄, Li₂PdBr₄, (NH₃)₂PdBr₄, H₂Pdl₄, Na₂Pdl₄, K₂Pdl₄, Li₂Pdl₄, (NH₃)₂Pdl₄, H₂Pd(NO₃)₄, Na₂Pd(NO₃)₄, K₂Pd(NO₃)₄, Li₂Pd(NO₃)₄, (NH₃)₂Pd(NO₃)₄, H₂Pd(OH)₄, Na₂Pd(OH)₄, K₂Pd(OH)₄, Li₂Pd(OH)₄, (NH₃)₂Pd(OH)₄, H₂Pd(CN)₄, Na₂Pd(CN)₄, K₂Pd(CN)₄, Li₂Pd(CN)₄, (NH₃)₂Pd(CN)₄;
(vii) M₂PdX₆ wherein M is H, Li, Na, K or NH₃ and X is Cl, Br, I, NO₃, OH or CN.
   Examples include but are not limited to H₂PdCl₆, Na₂PdCl₆, K₂PdCl₆, Li₂PdCl₆, (NH₃)₂PdCl₆, H₂PdBr₆, Na₂PdBr₆, K₂PdBr₆, Li₂PdBr₆, (NH₃)₂PdBr₆, H₂Pdl₆, Na₂Pdl₆, K₂Pdl₆, Li₂Pdl₆, (NH₃)₂Pdl₆, H₂Pd(NO₃)₆, Na₂Pd(NO₃)₆, K₂Pd(NO₃)₆, Li₂Pd(NO₃)₆, (NH₃)₂Pd(NO₃)₆, H₂Pd(OH)₆, Na₂Pd(OH)₆, K₂Pd(OH)₆, Li₂Pd(OH)₆, (NH₃)₂Pd(OH)₆, H₂Pd(CN)₆, Na₂Pd(CN)₆, K₂Pd(CN)₆, Li₂Pd(CN)₆, (NH₃)₂Pd(CN)₆;
(viii) PdX₂ wherein X is Cl, Br, I, NO₃, OH or CN.
   Examples include but are not limited to PdCl₂, PdBr₂, Pdl₂, Pd(NO₃)₂, Pd(NO₃)₂.xH₂O, Pd(OH)₂, Pd(CN)₂;
(ix) MAuX₄ wherein M is H, Li, Na or K and X is Cl, Br or I.
   Examples include but are not limited to HAuCl₄, HAuCl₄.3H₂O, HAuBr₄, HAul₄, LiAuCl₄, LiAuBr₄, LiAul₄, NaAuCl₄, NaAuBr₄, NaAul₄, KAuCl₄, KAuCl₄.xH₂O, KAuBr₄, KAuBr₄.2H₂O, KAul₄.
(x) AuX₃ wherein X is OAc, Cl, Br, I or OH.
   Examples include but are not limited to Au(OAc)₃, AuCl₃, AuBr₃, Aul₃, Au(OH)₃;
(xi) AuX wherein X is Cl, Br, I or CN.
   Examples include but are not limited to AuCl, AuBr, Aul, AuCN;
(xii) RhX₃ wherein X is Cl, Br, I or NO₃.
   Examples include but are not limited to RhCl₃, RhCl₃.xH₂O, RhBr₃, RhBr₃.xH₂O, Rhl₃, Rh(NO₃)₂;
(xiii) RuX₃ wherein X is Cl, Br or I.
   Examples include but are not limited to RuCl₃, RuCl₃.xH₂O, RuBr₃, RuBr₃.xH₂O, Rul₃;
(xiv) NiX₂ wherein X is F, Cl, Br, I, OH, OAc or NO₃.
   Examples include but are not limited to NiF₂, NiF₂.4H₂O, NiCl₂, NiCl₂.6H₂O, NiCl₂.xH₂O, NiBr₂, NiBr₂.3H₂O, Nil₂, Ni(OH)₂, Ni(OAc)₂, Ni(OAc)₂.4H₂O, Ni(OAc)₂.xH₂O, Ni(NO₃)₂, Ni(NO₃)₂.6H₂O; and
(xv) Pd(oxalate), Ni(oxalate), Ni(oxalate).2H₂O, [Rh(OAc)₂]₂, NiCO₃, Ni₃(citrate)₂.xH₂O.

Preferably, the at least one water soluble metal salt is selected from the group consisting of H₂PtCl₆, H₂PdCl₆, HAuCl₄, Na₂PdCl₄ and a combination thereof.

Preferably, the aqueous solvent is deionised water. Optionally, the water may further comprise one or more water miscible solvents. Typical water miscible solvents include but are not limited to alcohols (such as methanol, ethanol, n-propanol and/or iso-propanol), acetone, acetonitrile, dioxane, tetrahydrofuran, dimethylformamide and dimethylsulfoxide.

In one embodiment, the at least one amine is soluble in the aqueous solvent. If it is found, however, that the at least one amine is not soluble, the at least one amine may be further treated in order to solubilise it. For example, when the at least one amine is an amino acid which is not soluble in water, the amino acid may be solubilised by the addition of a base (such as an alkali hydroxide), an acid (e.g. hydrochloric acid) or a solvent (such as acetone).

The support, the at least one water-soluble metal salt and the at least one amine may be combined in the aqueous solvent in any suitable order. In one preferred process of the invention, however, the support, the at least one metal salt and the at least one amine are each mixed in a portion of aqueous solvent (e.g. water). The mixture of the at least one metal salt, followed by the mixture of the at least one amine, is then added to the mixture of the support. If desired, each aqueous mixture may be allowed to stand before stirring and, if desired, allowed to stand and re-stirred before combining them together optionally with further stirring.

In one embodiment, the mixture of the support is stirred and boiled before the mixtures of the at least one metal salt and at least one amine are added.

In one embodiment, the mixture of the at least one amine is added slowly (e.g. dropwise) to the mixture of the at least one metal salt and support.

In one embodiment, the mixture of the at least one metal salt is added rapidly to the mixture of the support.

In one embodiment, the mixture of the at least one amine is added rapidly to the mixture of the at least one metal salt and support.

In another preferred process of the invention, the at least one water-soluble metal salt and the at least one amine are reacted to form an amino acid metal complex which may be optionally recovered and purified. The amino acid metal complex may then be combined with the at least one support and the reaction mixture optionally heated.

Preferably, the reducing agent is (i) a combination of a base and formaldehyde, (ii) a formate, (iii) a borohydride, (iv) a hypophosphite, (v) hydrazine, or (vi) hydrogen.

When the reducing agent is a combination of a base and formaldehyde, the base can be an alkali metal hydroxide, an alkaline earth metal hydroxide, an alkali metal carbonate, an alkaline earth metal carbonate or an alkali metal hydrogen carbonate. Preferably, the base is sodium hydroxide, potassium hydroxide, magnesium hydroxide, calcium hydroxide, sodium hydrogen carbonate or potassium hydrogen carbonate, especially sodium hydroxide or sodium hydrogen carbonate.

The combination of a base and formaldehyde can be added to the reaction mixture to form a formate in situ. Alternatively, the formate may be prepared prior to its addition to the support, at least one water soluble metal salt and at least one amine. In this instance, when the reducing agent is a formate, the formate may be an alkali metal formate or alkaline earth metal formate. Examples of formates are sodium formate, potassium formate, magnesium formate and calcium formate.

When the reducing agent is a borohydride, the borohydride may be an alkali metal borohydride, such as sodium borohydride or potassium borohydride. Preferably, the alkali metal borohydride is sodium borohydride.

When the reducing agent is a hypophosphite, the hypophosphite can be an alkali metal hypophosphite. Examples of alkali metal hypophosphites are sodium hypophosphite and potassium hypophosphite. Sodium hypophosphite is preferred.

When the reducing agent is hydrazine, the hydrazine can be anhydrous or a solution in a solvent, such as tetrahydrofuran or water.

The reducing agents described above may be added to the aqueous mixture of the support, the at least one water soluble metal salt and the at least one amine alone or as a solution in further aqueous solvent.

When the reducing agent is hydrogen, the supported metal catalysts may be reduced prior to or during a hydrogenation reaction.

Step (a) and step (b) may be carried out at one or more temperatures between about 15°C and 100°C. In one embodiment, step (a) is carried out at a temperature between about 15°C and about 25°C, most preferably room temperature. In another embodiment, step (a) is carried out at the boiling temperature of the aqueous solvent.

In one embodiment, step (b) is carried out at one or more temperatures between about 25°C and about 100°C. In a preferred embodiment, after the addition of the reducing agent, the reaction mixture is heated and stirred to about 90°C. When the reaction mixture reaches this temperature, the reaction mixture is allowed to cool, for example by removing the heat source or adding further aqueous solvent, to about 60°C or below, whereupon the stirring may be stopped if desired. In another preferred embodiment, after the addition of the reducing agent, the reaction mixture is boiled for a period of time and then cooled to about 60°C or below e.g. by the addition of further solvent or removing the heat source.

The reaction may be continued for a period of from about 30 minutes to several hours, but is normally complete within about four hours. On completion, the supported metal catalyst may be collected and separated from the reaction mixture by any conventional separation technique such as filtration, decantation or centrifugation, then subjected to further purification or processing steps such as washing and/or drying if so desired. The separated reaction mixture may be further treated to recover additional supported metal catalyst.

The supported metal catalyst may be prepared on any desired scale. For example, it has been found that the above mentioned process may be reliably scaled up to prepare about 3kg of supported metal catalyst.

### Hydrogenation Reactions

The supported metal catalysts of the present invention may be used in hydrogenation reactions. For example, it has been found that the catalysts are chemoselective in the hydrogenation of substrates containing one or more halogen atoms i.e. the desired hydrogenation reaction proceeds with a reduced propensity for concurrent dehalogenation. Without wishing to be bound by theory, it is believed that the at least one amine binds to the surface of the catalyst and that the presence of the at least one amine controls the size and shape of the catalyst which subtly influences the surface of the catalyst electronically. Another aspect of the invention therefore provides a process for the preparation of an optionally substituted amine, comprising the step of hydrogenating an optionally substituted benzyl-amine in the presence of hydrogen and a supported metal catalyst as described herein.

In one embodiment, the benzyl-amine is a compound of formula A, which on hydrogenation forms a compound of formula B and C: 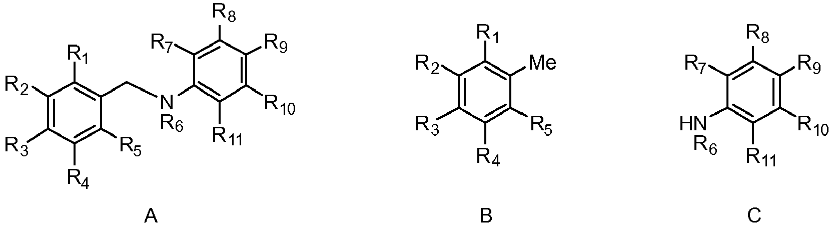 wherein,
R₁, R₂, R₃, R₄, R₅, R₆, R₇, R₈, R₉, R₁₀ and R₁₁ are independently selected from the group consisting of H, alkyl, aryl, alkenyl, alkynyl, arylalkyl-, -O-alkyl, -O-aryl, -O-alkylaryl, heterocycle, halo, -NO₂, -CN, -SCN, -NCS, -OH, -C(halo)₃, -NR'R"R"', -COR', -COOH,-COOR', -OCOR', -OC(O)-OR', -CONR'R", -C=N-O-R', -S-alkyl, -S-aryl, -S-alkylaryl, -SO₂R',-S(O)₂NR'R", -O-S(O)-R', -C(S)R', -C(S)OH, -C(S)OR', -OC(S)-OR', -C(S)NR'R", wherein the alkyl, aryl, alkenyl, alkynyl, arylalkyl- and heterocyclic groups may be optionally further substituted; and
R', R" and R'" are independently selected from the group consisting of H, alkyl, aryl, arylalkyl- and heterocycle, wherein the alkyl, aryl, arylalkyl- and heterocyclic groups may be optionally further substituted.

In one embodiment, R₁, R₂, R₃, R₄ and R₅ are each H.

In one embodiment, at least one of R₇, R₈, R₉, R₁₀ or R₁₁ is a halo group, especially -Cl.

In one embodiment, at least one of R₉, R₁₀ or R₁₁ is a halo group.

In one embodiment, the process further comprises an acid, for example, a mineral acid such as hydrochloric acid, hydrobromic acid or hydroiodic acid. The addition of an acid may be useful when the compound of formula A contains one or more halogen atoms. This is because the presence of the acid has been found to further reduce the undesirable dehalogenation reaction.

The molar ratio of acid to substrate may be any suitable molar ratio. Conveniently, the molar ratio may be about 1:1.

In yet another aspect, the present invention provides a process for the preparation of an optionally substituted arylamine, comprising the step of hydrogenating an optionally substituted aryl compound comprising one or more nitro groups in the presence of hydrogen and a supported metal catalyst as defined herein.

In one embodiment, the aryl compound further comprises one or more halo groups.

Preferably, the process further comprises an acid, for example, a mineral acid such as hydrochloric acid, hydrobromic acid or hydroiodic acid. The addition of the acid is useful for the same reason as given above i.e. the presence of the acid further reduces the undesirable dehalogenation reaction. The molar ratio of acid to substrate may also be as described above.

In yet another aspect, the present invention provides a process for the preparation of an optionally substituted alkene, comprising the step of hydrogenating an optionally substituted alkyne in the presence of hydrogen and a supported metal catalyst as defined herein.

Preferably, wherein the alkyne is a compound of formula D: 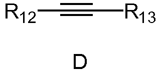 wherein,
R₁₂ and R₁₃ are independently selected from the group consisting of H, alkyl, aryl, alkenyl, alkynyl, arylalkyl-, -O-alkyl, -O-aryl, -O-alkylaryl, heterocycle, halo, -NO₂, -CN, -SCN, -NCS, - OH, -C(halo)₃, -NR'R"R"', -COR', -COOH, -COOR', -OCOR', -OC(O)-OR', -CONR'R", -C=NO-R', -S-alkyl, -S-aryl, -S-alkylaryl, -SO₂R', -S(O)₂NR'R", -O-S(O)-R', -C(S)R', -C(S)OH, - C(S)OR', -OC(S)-OR', -C(S)NR'R", wherein the alkyl, aryl, alkenyl, alkynyl, arylalkyl- and heterocyclic groups may be optionally further substituted; and
R', R" and R'" are independently selected from the group consisting of H, alkyl, aryl, arylalkyl- and heterocycle, wherein the alkyl, aryl, arylalkyl- and heterocyclic groups may be optionally further substituted.

The supported metal catalysts of the present invention are useful in the preparation of cis-alkenes, trans-alkenes or a mixture thereof. In one embodiment, the hydrogenation is selective. In a preferred embodiment, the alkene predominantly comprises a cis-alkene.

The hydrogen pressures of the hydrogenation reactions mentioned above are suitably in the range of up to about 100 bar and conveniently in the range of from about 1 to 10 bar.

Preferably, the ratio of catalyst : starting material may vary in the range from about 1:1 to about 1:20,000, more preferably, 1:1 to about 1:3000, even more preferably, about 1:200 to about 1:2500 and most preferably, about 1:250 to about 1:2000.

The hydrogenation reactions preferably further comprise a solvent. Any suitable solvent may be used, for example, aqueous solvents, polar solvents, non-polar solvents, aprotic solvents, protic solvents or a combination thereof. Preferably, the solvent is one or more C₁₋₁₀ alkanols, more preferably, methanol, ethanol, propanol isomers (i.e. n- or i-propanol), butanol isomers (i.e. n-, i- or t-butanol), pentanol isomers, hexanol isomers, heptanol isomers or combinations thereof. Methanol and ethanol are especially preferred solvents.

The concentration of the starting material in the solvent may be any suitable concentration. Conveniently, the concentration may be about 0.5M.

Reaction temperatures are suitably in the range from 10 to 100°C, preferably in the range from about 15 to about 80°C, most preferably about 20 to about 60°C.

The reactants may be added in any suitable order, but in a preferred process of the invention, the starting material (i.e. substrate to be hydrogenated) and the supported metal catalyst is placed in a hydrogenation vessel, together with a solvent (if used). The vessel is then charged with hydrogen and heated and/or stirred if necessary. The reaction may be continued until the calculated number of moles of hydrogen has been consumed.

The invention will now be described by way of example only and with reference to the following drawings in which:
Figures 1a-c are TEM analyses of Pd-Lysine on a carbon support.
Figure 2 is a XRD of Pd-Lysine on a carbon support.
Figure 3 illustrates the XPS analysis of Pd-Gly / GSX catalyst.
Figure 4 is a XRD of 5% Pd-Pro / GSX catalyst.
Figure 5 is a XRD of 5% Pd-Ala / GSX catalyst.
Figure 6 is a XRD of 5% Pd-Arg / GSX catalyst.
Figure 7 is a XRD of 5% Pd-Ser / GSX catalyst.
Figure 8 is a XRD of 5% Pd-D-Phe / GSX catalyst.
Figure 9 is a XRD of 5% Pd-L-Phe / GSX catalyst.
Figure 10 is a XRD of 10% Pd-Gly / GSX catalyst.
Figure 11 is a XRD of 1 % Pd-Gly / GSX catalyst.
Figure 12 is a XRD of 5% Pd-Gly / Alumina catalyst.
Figure 13 is a XRD of 5% Pd / Alumina catalyst (comparative).
Figures 14a-b are the TEM analyses of 0.5% Pd-Gly / Graphite catalyst.
Figure 15 illustrates the XPS analysis of 0.5% Pd-Gly / Graphite catalyst.
Figure 16 is a XRD of Au-Pd-Lys / GSX catalyst (1:1 wt% with respect to each metal).
Figure 17 is the TEM analysis of a Au-Pd-Lys / GSX catalyst (1:1 wt% with respect to each metal).
Figure 18 illustrates an analysis of lysine-precipitated Pd / C catalyst examined over different time periods in an N-debenzylation reaction both in the presence (LysH) and absence (Lys) of 1 eq. HCl.
Figure 19 illustrates an analysis Pd/C catalyst (Type 39) examined over different time periods in an N-debenzylation reaction both in the presence (39H) and absence (39) of 1 eq. HCl (comparative).
Figure 20 illustrates an analysis of various catalysts in an N-debenzylation reaction.
Figure 21 illustrates the hydrogen uptake curves of 5% Pd-lysine/C, 5% Pd/C (Type 39) and 2.5% Pd-2.5% Au-Lysine/C in the hydrogenation of 2-chloronitrobenzene.
Figure 22 illustrates the hydrogen uptake curves of Lindlar catalyst, Pd/C (Type 39) and Pd-Gly / T44 during the hydrogenation of 3-hexyn-1-ol.

### Examples

The ligands butylamine, hexylamine, 6-amino caproic acid, 1,6-diaminohexane, lysine, glycine, alanine, arginine, serine, proline, asparagines, aspartic acid, valine and phenylalanine were purchased from Alfa Aesar. The precious metal salts (Na₂PdCl₄, H₂PtCl₆, HAuCl₄) were purchased from Alfa Aesar. The supports Carbon GSX was purchased from Norit, L4S, 2S and CPL were purchased from Ceca. T44 Graphite was purchased from Timcal and calcium carbonate "Calopake F" was purchased from Ellis & Everard. Pd on alumina and Pd/C (5% Pd/C Type 39, 5% Pd/C 87L, 5% Pd/Alumina Type 324, 5% PdPb/CaCO₃ A305060-5, and 5% Pt/C type 18) were obtained from Johnson Matthey PLC.

### Example 1

### Preparation of Palladium Supported Catalysts

### The use of lysine to precipitate palladium onto carbon

Carbon GSX (9.5g) was weighed out and 60ml of DI water was added to it and briefly stirred. The slurry was then allowed to stand for 1 hour. After this time, the stirring was resumed. Na₂PdCl₄ (1.46g; (34.97% solid) 0.51g of metal) was dissolved in water (8ml) and added to the slurry. This was stirred for 30 minutes. Lysine (0.77g, 1 equiv, dissolved in 10 ml of water) was then added dropwise to the slurry over 5 minutes. It was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

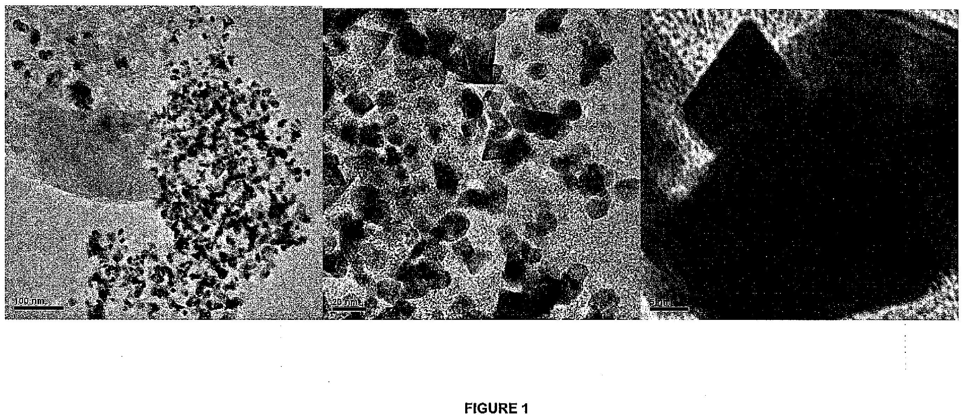

The TEM analysis of the catalyst is illustrated in Figures 1a-c (mean particle size = 28.1nm, standard deviation = 20.0nm, minimum = 6.7nm, maximum = 112.8nm).

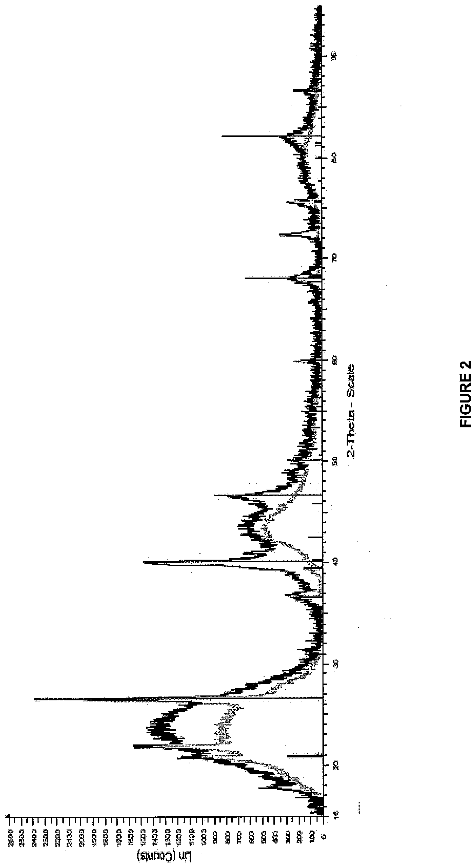

The diffraction pattern Figure 2 indicates the presence of a significant amount of poorly crystalline Palladium (Pd, PDF No.00-046-1043). Rietveld analysis estimates the Palladium crystallite size to be 15.8nm. A standard GSX carbon pattern has been included in Figure 2 for reference. This standard GSX pattern shows the presence of SiO2 - Quartz (SiO2, PDF No.00-046-1045) also evident in the sample.

The above Example has since been scaled up from 10g scale to a 100g and a 3kg scale.

### Example 2

### The Use of Different Carbon Supports

The reaction as exemplified in Example 1 has also undertaken with different carbon supports - Ceca L4S, Ceca 2S, CPL. Large particles were similarly formed.

### Example 3

### The Use of Other Ligands to Precipitate Palladium onto Carbon

The reaction exemplified in Example 1 has been undertaken using different ligands.

### a. Butylamine Precipitation

Carbon GSX (9.5g) was weighed out and 60ml of DI water was added to it and briefly stirred. The slurry was then allowed to stand for 1 hour. After this time, the stirring was resumed. Butylamine (0.34g, 1 equiv, dissolved in 5 ml of water) was then added to the slurry. It was then stirred for 30 minutes. Na₂PdCl₄ (1.46g; (34.97% solid) 0.51g of metal) was dissolved in water (25ml) and added dropwise to the slurry over 10 minutes. This was stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the solution was then filtered and the resulting black solid was washed with 500ml of DI water.

The black solid was collected and dried in the oven at 90°C for 24 hours.

The XRD diffraction pattern indicates the presence of a major amount of poorly crystalline Palladium (Pd, PDF No.00-046-1043) supported on carbon. The Pd crystallite size was estimated by Rietveld analysis (LVol-IB method) to be 7.5nm.

### b. 6-Amino caproic acid precipitation

Carbon GSX (9.5g) was weighed out and 60ml of DI water was added to it and briefly stirred. The slurry was then allowed to stand for 1 hour. After this time, the stirring was resumed. Na₂PdCl₄ (1.46g; (34.97% solid) 0.51g of metal) was dissolved in water (8ml) and added to the slurry. This was stirred for 30 minutes. 6-Amino caproic acid (0.62g, 1 equiv, dissolved in 10 ml of water) was then added dropwise to the slurry over 5 minutes. It was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

The XRD diffraction pattern indicates the presence of a major amount of poorly crystalline Palladium (Pd, PDF No.00-046-1043) supported on carbon. The Pd crystallite size has been estimated by Rietveld analysis (LVol-IB method) to be ~8.6nm.

### c. 1,6-diaminohexane

The same synthetic method was used as above with the exception that one equivalent of 1,6-diaminohexane (0.55g) was used to precipitate the metal onto the carbon support.

The XRD diffraction pattern indicates the presence of a significant amount of poorly crystalline Palladium (Pd, PDF No.00-046-1043) supported on carbon. The Pd crystallite size has been estimated by Rietveld analysis (LVol-IB method) to be ~11.9nm.

### d. Glycine

The same synthetic method was used as above with the exception that one equivalent of Glycine (0.35g) was used to precipitate the metal onto the carbon support.

The XRD diffraction pattern indicates the presence of a major amount of poorly crystalline Palladium (Pd, PDF No.00-046-1043) supported on carbon. The Pd crystallite size has been estimated by Rietveld analysis (LVol-IB method) to be -16.9nm.

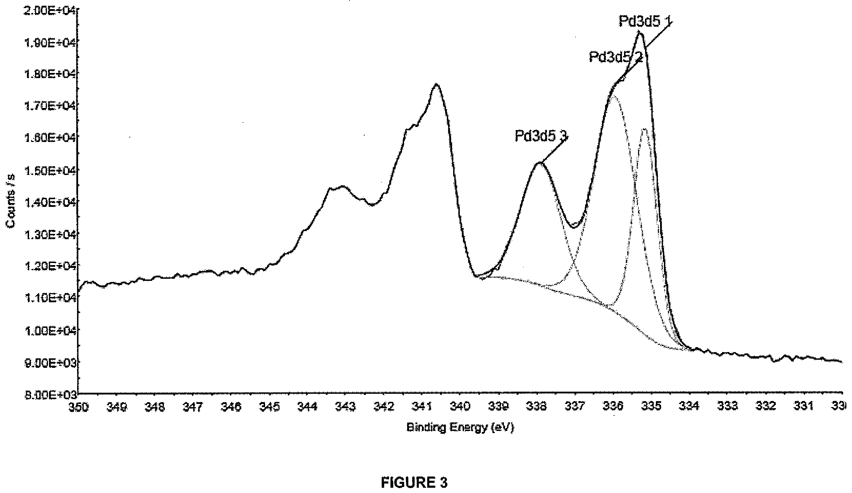

XPS analysis of the Pd-Gly / GSX catalyst formed is illustrated in Figure 3. Examination of the XPS spectra indicates that the amino acid remains bound on the surface. In addition to metallic Pd and oxidic PdO peaks that are seen in conventional Pd/C catalysts (Pd3d5 1 and Pd3d5 3 peaks in the spectra respectively), a significant peak with intermediate binding energy is also observed (Pd3d5 2). This has been assigned to amino-palladium bonding and illustrates that the presence of the amino acid subtly modifies the surface of the catalyst electronically.

### e. Hexylamine

The same synthetic method was used (as above) with the exception that one equivalent of hexylamine (0.47g) was used to precipitate the metal onto the carbon support.

The XRD diffraction pattern indicates the presence of a major amount of poorly crystalline Palladium (Pd, PDF No.00-046-1043) supported on carbon. The Pd crystallite size has been estimated by Rietveld analysis (LVol-IB method) to be ~7.3nm.

### Example 4

### Use of Different Amino Acids in Pd/C preparation

Carbon GSX (9.5g) was weighed out and 60ml of DI water was added to it and briefly stirred. The slurry was then allowed to stand for 1 hour. After this time, the stirring was resumed. Na₂PdCl₄ (1.46g; (34.97% solid) 0.51g of metal) was dissolved in water (8ml) and added to the slurry. This was stirred for 30 minutes. The relevant amino acid (1 equiv, dissolved in 10 ml of water) was then added to the slurry. It was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle and DI water was added to the slurry until the temperature had fallen to below 60°C, the stirring was also stopped. The solution was then filtered and the resulting black solid was washed with 500ml of DI water.

The black solid was collected and dried in the oven at 90°C for 24 hours.

The following amino acids were used:
a) Proline (0.55g)
b) Alanine (0.43g)
c) Arginine (0.83g)
d) Serine (0.50g)

The use of other amino acids were also tried (namely: asparginine, aspartic acid, valine and phenylalanine). These did not dissolved in water at pH 7, therefore the above preparation could not be used in this instance. However, the addition of base did dissolve the amino acids (due to deprotonation of the carboxylic acid group). The preparation of phenylalanine stabilised Pd/C was attempted using one equivalent of NaOH to solubilise the amino acid.

In order to see if the use of different isomers of amino acids had any effect, the D- (Sample e) and L- (Sample f) isomers of phenylalanine were used.
e) D-Phenylalanine (0.79g)
f) L-Phenylalanine (0.79g)

0.79g of each of the isomers were combined with 0.19g of NaOH (1.0 equivalent) and stirred in 10ml of DI water.

### a. 5% Pd-Pro / GSX

The same preparation as above was used.

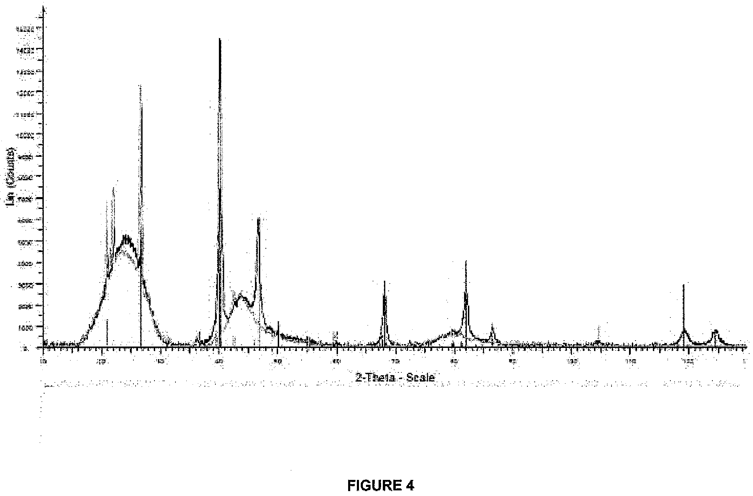

The diffraction pattern Figure 4 indicates the presence of a major amount of poorly crystalline Palladium (Pd, PDF No.00-046-1043) supported on carbon. The Pd crystallite size has been estimated by Rietveld analysis (LVol-IB method) to be ~13.3nm.

### b. 5% Pd-Ala / GSX

The same preparation as above was used.

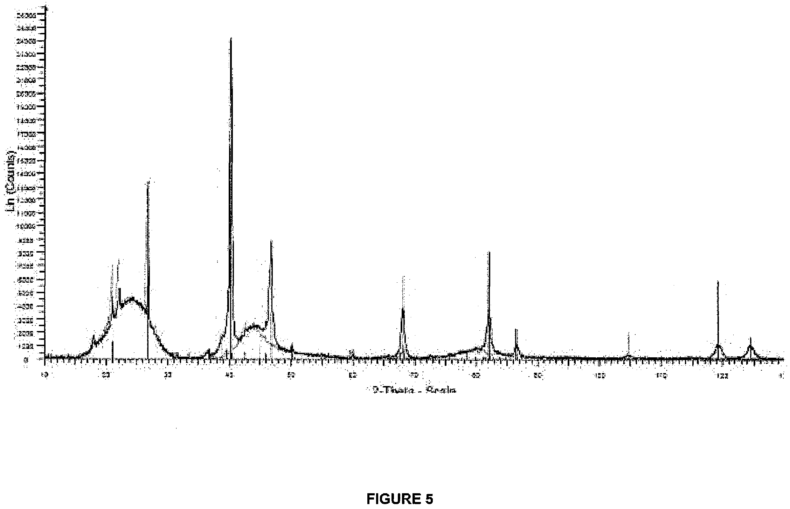

The diffraction pattern Figure 5 indicates the presence of a major amount of poorly crystalline Palladium (Pd, PDF No.00-046-1043) supported on carbon. The Pd crystallite size has been estimated by Rietveld analysis (LVol-IB method) to be ~17.4nm.

### c. 5% Pd-Arg / GSX

The same preparation as above was used.

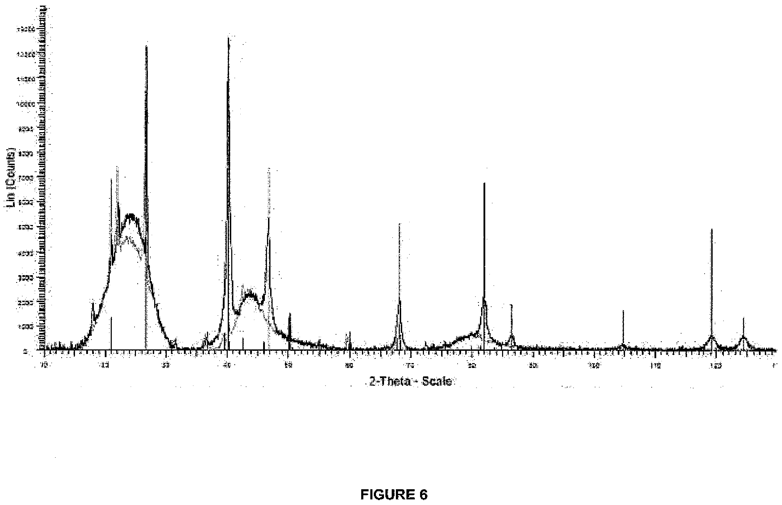

The diffraction pattern Figure 6 indicates the presence of a significant amount of poorly crystalline Palladium (Pd, PDF No.00-046-1043) supported on carbon. The Pd crystallite size has been estimated by Rietveld analysis (LVol-IB method) to be ~11.2nm.

### d. 5% Pd-Ser GSX

The same preparation as above was used.

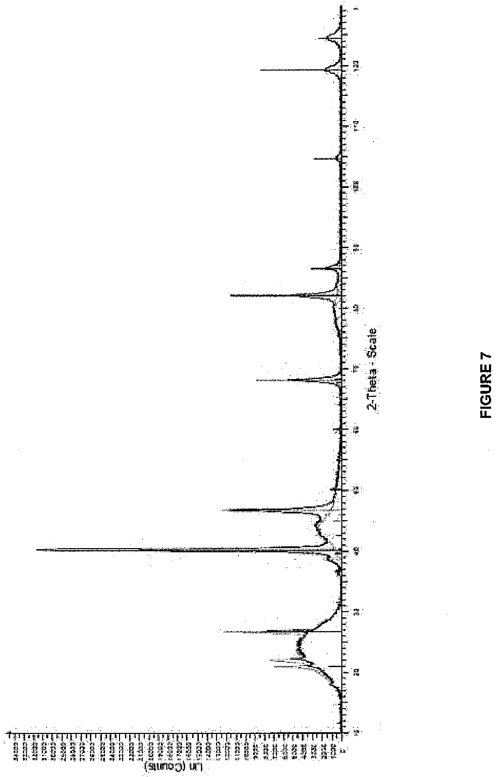

The diffraction pattern Figure 7 indicates the presence of a major amount of poorly crystalline Palladium (Pd, PDF No.00-046-1043) supported on carbon. The Pd crystallite size has been estimated by Rietveld analysis (LVol-IB method) to be ~16.7nm.

### e. 5% Pd-D-Phe / GSX

The same preparation as above was used.

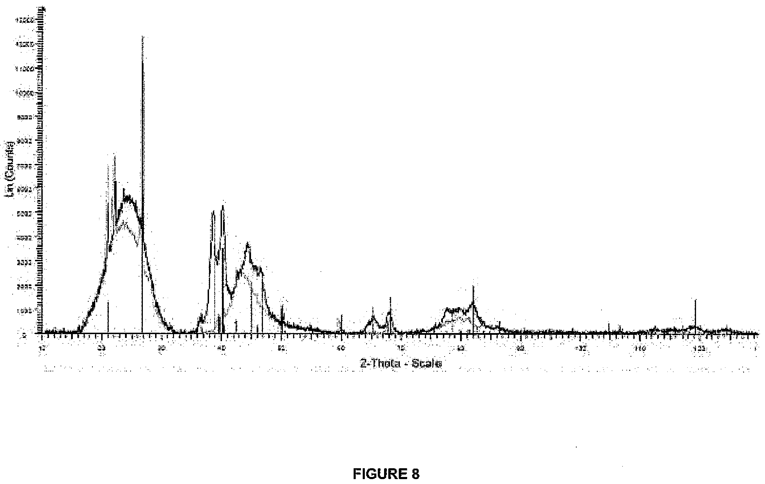

The diffraction pattern Figure 8 indicates the presence of a major amount of poorly crystalline Palladium (Pd, PDF No.00-046-1043) supported on carbon. The Pd crystallite size has been estimated by Rietveld analysis (LVol-IB method) to be ~5.7nm.

### f. 5% Pd-L-Phe / GSX

The same preparation as above was used.

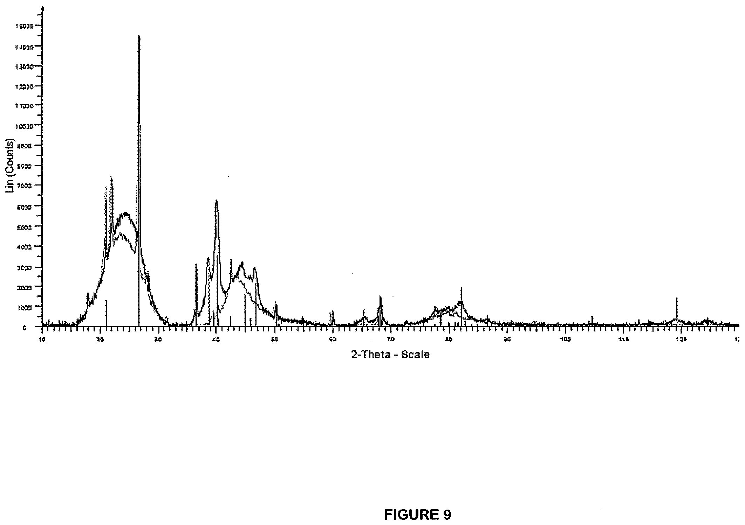

The diffraction pattern Figure 9 indicates the presence of a significant amount of poorly crystalline Palladium (Pd, PDF No.00-046-1043) supported on carbon. The Pd crystallite size has been estimated by Rietveld analysis (LVol-IB method) to be ~7.9nm.

### Example 5

### The Use of Alternative Reducing Agents

### a. Sodium Borohydride Reduction

Carbon GSX (9.5g) was weighed out and 60ml of DI water was added to it and briefly stirred. The slurry was then allowed to stand for 1 hour. After this time, the stirring was resumed.

Na₂PdCl₄ (1.46g; (34.97% solid) 0.51g of metal) was dissolved in water (8ml) and added to the slurry. This was stirred for 30 minutes. Lysine, 0.77g, 1 equiv, dissolved in 10 ml of water) was then added dropwise to the slurry over 5 minutes. It was then stirred for a further 30 minutes.

NaBH₄ (0.20g) was dissolved in DI water (6ml). This solution was then added to the slurry and stirred for 1 hour. The flask was then allowed to settle. The solution was then filtered and the resulting black solid was washed with 500ml of DI water. The black solid was collected and dried in the oven at 90°C for 24 hours.

The XRD pattern indicates the presence of a significant amount of poorly crystalline Palladium (Pd, PDF No.00-046-1043) supported on carbon. The Pd crystallite size has been estimated by Rietveld analysis (LVol-IB method) to be ~6.6nm.

### b. Sodium Hypophosphite Reduction

The same synthetic method was used as above with the exception that one equivalent of NaH₂PO₂ (0.50g) was used as the reductant.

The XRD pattern indicates the presence of a major amount of poorly crystalline Palladium (Pd, PDF No.00-046-1043) supported on carbon. The Pd crystallite size has been estimated by Rietveld analysis (LVol-IB method) to be ~5.9nm.

The use of sodium hypophosphite also results in a poorer monodispersity of metal particles on the surface, in comparison to formaldehyde.

### Example 6

### Varying the Amounts of Ligand Precipitation reaction

### a. 2.0 Equivalents of Lysine

Carbon GSX (9.5g) was weighed out and 60ml of DI water was added to it and briefly stirred. The slurry was then allowed to stand for 1 hour. After this time, the stirring was resumed. Na₂PdCl₄ (1.46g; (34.97% solid) 0.51g of metal) was dissolved in water (8ml) and added to the slurry. This was stirred for 30 minutes.

Lysine (1.54g, 2 equivs, dissolved in 10 ml of water) was then added dropwise to the slurry over 5 minutes. It was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

### b. 0.1 Equivalents of Lysine

The same synthetic method was used as above with the exception that 0.1 equivalents of lysine (0.08g) was used to precipitate the metal.

### Example 7

### Glycine-precipitated Pd/GSX Catalysts of Different Metal Loadings

### a. 10% Pd Loading

Carbon GSX (9.0g) was weighed out and 60ml of DI water was added to it and briefly stirred. The slurry was then allowed to stand for 1 hour. After this time, the stirring was resumed.

Na₂PdCl₄ (2.92g; (34.97% solid) 0.51g of metal) was dissolved in water (8ml) and added to the slurry. This was stirred for 30 minutes.

Glycine (0.70g, 1 equiv, dissolved in 10 ml of water) was then added to the slurry. It was then stirred for a further 30 minutes.

NaOH (0.62g) was dissolved in DI water (6ml) and 2.04ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

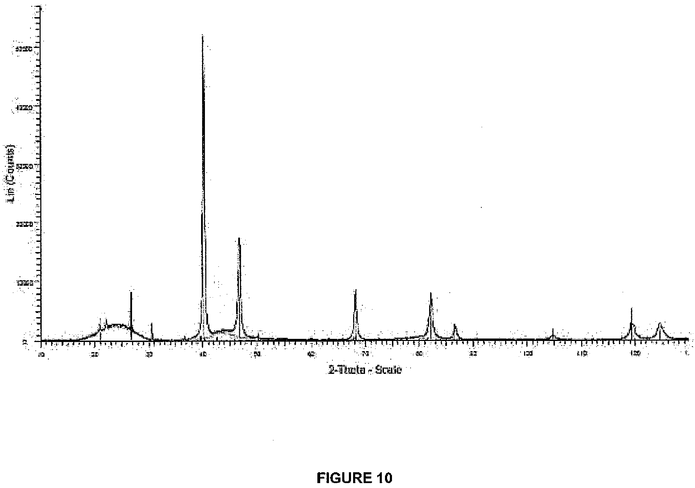

The diffraction pattern Figure 10 indicates the presence of a major amount of poorly crystalline Palladium (Pd, PDF No.00-046-1043) supported on carbon. The Pd crystallite size has been estimated by Rietveld analysis (LVol-IB method) to be ~19.6nm.

### b. 1% Pd Loading

Carbon GSX (9.9g) was weighed out and 60ml of DI water was added to it and briefly stirred. The slurry was then allowed to stand for 1 hour. After this time, the stirring was resumed.

Na₂PdCl₄ (0.29g; (34.97% solid) 0.51g of metal) was dissolved in water (8ml) and added to the slurry. This was stirred for 30 minutes.

Glycine (0.07g, 1 equiv, dissolved in 10 ml of water) was then added to the slurry. It was then stirred for a further 30 minutes.

NaOH (0.06g) was dissolved in DI water (6ml) and 0.20ml of formaldehyde solution (40%) was added to the base and stirred. This solution was then added to the slurry. this was then heated up to 90°C. Upon reaching this temperature, the flask was raised from the heating mantle. When the temperature had fallen to below 60C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

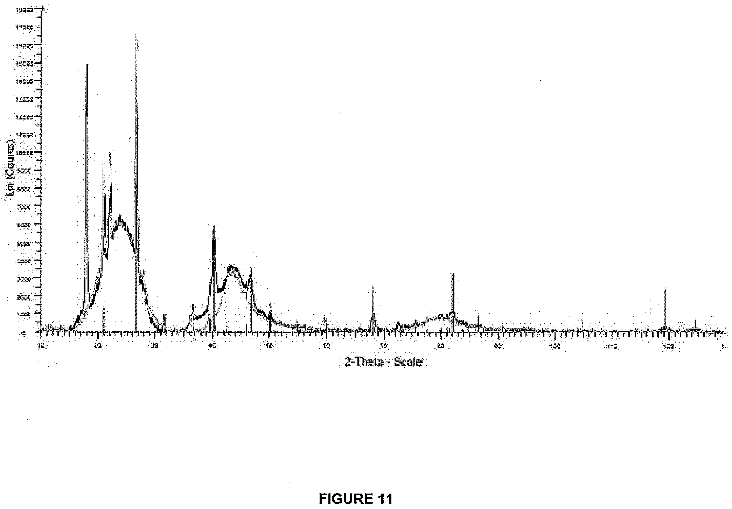

The diffraction pattern Figure 11 indicates the presence of a significant amount of poorly crystalline Palladium (Pd, PDF No.00-046-1043) supported on carbon. The Pd crystallite size has been estimated by Rietveld analysis (LVol-IB method) to be ~9.4nm.

### Example 8

### Preparation of Pd on Alumina Catalysts

### a. Pd Glycine-Precipitated on Alumina (5% Pd-Gly / Alumina)

Alumina (9.5g) was weighed out and 60ml of DI water was added to it and briefly stirred. Na₂PdCl₄ (1.46g; (34.97% solid) 0.51g of metal) was dissolved in water (8ml) and added to the slurry. This was stirred for 30 minutes. Glycine (0.35g, 1 equiv, dissolved in 10 ml of water) was then added to the slurry. It was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle and DI water was added to the slurry until the temperature had fallen to below 60°C, the stirring was also stopped. The solution was then filtered and the resulting solid was washed with 500ml of DI water. The filtrate had a faint tinge of yellow. The dark brown solid was collected and dried in the oven at 90°C for 24 hours.

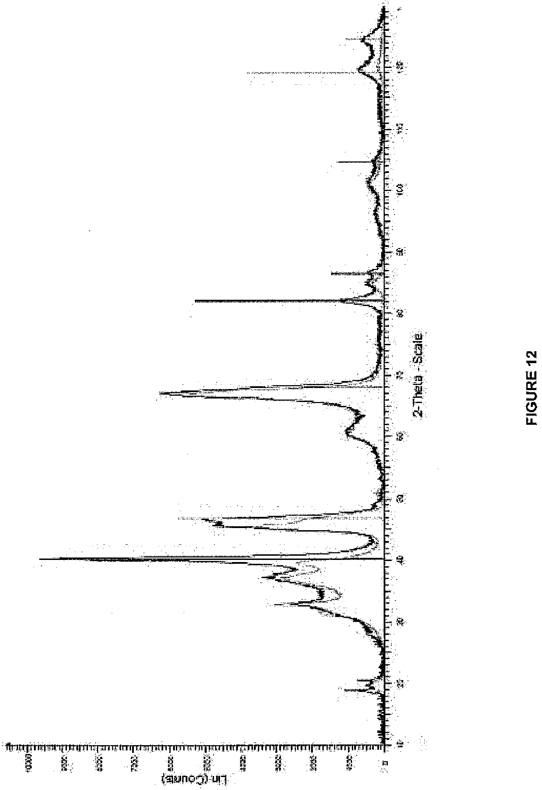

The diffraction pattern Figure 12 indicates the presence of a major amount of poorly crystalline Palladium (Pd, PDF No.00-046-1043) supported on delta alumina. The Pd crystallite size has been estimated by Rietveld analysis (LVoI-IB method) to be -8.6nm.

### b. Pd on Alumina - No precipitant (5% Pd / Alumina) (Comparative)

Alumina (9.5g) was weighed out and 60ml of DI water was added to it and briefly stirred. Na₂PdCl₄ (1.46g; (34.97% solid) 0.51g of metal) was dissolved in water (8ml) and added to the slurry. This was stirred for 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle and DI water was added to the slurry until the temperature had fallen to below 60°C, the stirring was also stopped. The solution was then filtered and the resulting solid was washed with 500ml of DI water.

The dark brown solid was collected and dried in the oven at 90°C for 24 hours.

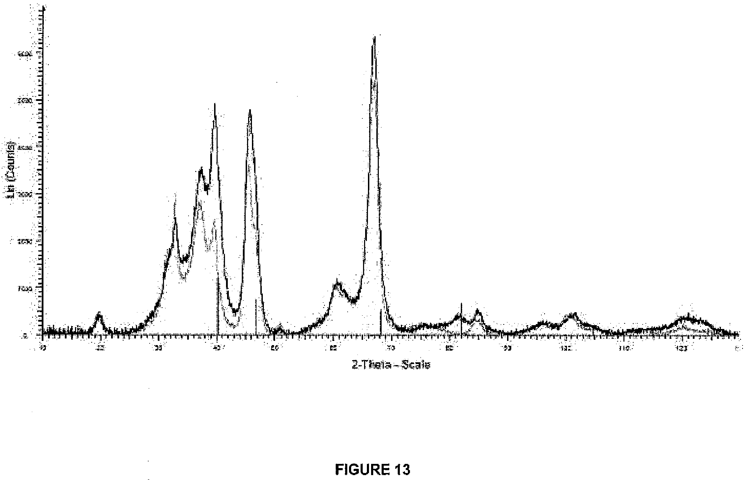

The diffraction pattern Figure 13 indicates the presence of a minor amount of poorly crystalline Palladium (Pd, PDF No.00-046-1043) supported on delta alumina. The Pd crystallite size has been estimated by Rietveld analysis (LVol-IB method) to be ~2.6nm.

### Example 9

### Preparation of Platinum supported catalysts

### Lysine-Modified Pt/C

Carbon GSX (9.5g) was weighed out and 60ml of DI water was added to it. The slurry was then stirred and boiled for 30 minutes.

H₂PtCl₆ (2.04g; (25.0% solution) 0.51g of metal) was dissolved in water (8ml) and rapidly added to the boiling slurry. This was boiled for a further 30 minutes. Lysine (0.38g, 2.61 mmol, 1 equiv, dissolved in 10 ml of water) was then added rapidly, which was then boiled for a further 90 minutes.

0.36ml of formaldehyde solution (40%) was dissolved in 5 ml of water along with one equimolar quantity of NaHCO₃ (0.44g, 5.18mmol) and added rapidly to the slurry. The boiling slurry was maintained for a further one hour. After this time, the slurry was topped up with water in order to cool it to less than 60°C.

After standing, the supernatant was decanted off and the black solid was collected by filtration. This was then washed with 500ml of DI water. The black solid was collected and dried in the oven at 90°C.

### ICP Analysis 5.15% Pt

The diffraction pattern (not shown) indicated the presence of a major amount of crystalline platinum supported on GSX carbon. The Pt crystallite size was estimated by Rietveld analysis (LVoI-IB method) to be ~9.0nm.

### Example 10

### Preparation of Gold Supported Catalysts

Carbon GSX (9.5g) was weighed out and 60ml of DI water was added to it and briefly stirred. The slurry was then allowed to stand for 1 hour. After this time, the stirring was resumed.

Hydrogen tetrachloroaurate (1.20g; (41.24% solution) 0.51g of metal) was dissolved in water (8ml) and added to the slurry. This was stirred for 30 minutes.

Lysine 0.38g (1 equiv, dissolved in 10 ml of water) was then added immediately to the slurry, which was then stirred for a further 30 minutes.

0.63ml of formaldehyde solution (40%, 3.5 molar equivalents) was added to the slurry and stirred for 30 minutes. This was then heated up to reflux and maintained at this temperature for a further 30 minutes. The solution was then allowed to cool to room temperature, filtered and the resulting black solid was washed with 500ml of DI water.

The black solid was collected and dried in the oven at 90°C for 24 hours.

### ICP Analysis 5.34% Au

The diffraction pattern (not shown) indicated the presence of a major amount of crystalline Gold supported on GSX carbon. The Au crystallite size was estimated by Rietveld analysis (LVol-IB method) to be ~27.5nm.

### Example 11

### The use of peptides to precipitate palladium onto a support

### a. Gly-Gly, Diglycine

Carbon GSX (9.5g) was weighed out and 60ml of DI water was added to it and briefly stirred. The slurry was then allowed to stand for 1 hour. After this time, the stirring was resumed.

Na₂PdCl₄ (1.46g; (34.97% solid) 0.51g of metal) was dissolved in water (8ml) and added to the slurry. This was stirred for 30 minutes.

Diglycine (Gly-Gly) (0.63g, 4.79mmol, 1 equiv, dissolved in 10 ml of water) was then added immediately to the slurry, which was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

The diffraction pattern (not shown) indicated the presence of a major amount of crystalline palladium supported on GSX carbon. The Pd crystallite size has been estimated by Rietveld analysis (LVol-IB method) to be ~23.9nm.

### b. Gly-Gly-Gly, Triglycine

Carbon GSX (9.5g) was weighed out and 60ml of DI water was added to it and briefly stirred. The slurry was then allowed to stand for 1 hour. After this time, the stirring was resumed.

Na₂PdCl₄ (1.46g; (34.97% solid) 0.51g of metal) was dissolved in water (8ml) and added to the slurry. This was stirred for 30 minutes.

Triglycine (Gly-Gly-Gly) (0.92g, 4.79mmol, 1 equiv, dissolved in 10 ml of water) was then added immediately to the slurry, which was then stirred for a further 30 minutes.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

The diffraction pattern (not shown) indicated the presence of a major amount of crystalline palladium supported on GSX carbon. The Pd crystallite size has been estimated by Rietveld analysis (LVol-IB method) to be ~21.3nm.

### Example 12

A bis(glycinato) palladium(II) complex (Pd(gly)₂) was prepared according to the method described by J. S. Coe and J. R. Lyons in J. Chem. Soc. A, 1971, 829-33. This was then used as a precursor in the reaction where the amino acid was already complexed to the metal prior to addition.

Carbon GSX (9.5g) was weighed out and 60ml of DI water was added to it and briefly stirred. The slurry was then allowed to stand for 1 hour. After this time, the stirring was resumed.

Pd(gly)₂ (1.22g, 4.79mmol, 0.51g of metal) was dissolved in water (20ml) and added to the slurry. The complex was not that soluble and so had to be heated to get it into solution. Upon addition much of this was observed to precipitate out. This was stirred for 2 hours. After this time, there was no visible sign of any of the palladium precursor either in solution or as a solid. Without wishing to be bound by theory, it is believed that, in the additional solvent, it had dissolved and stuck to the carbon support.

NaOH (0.31g) was dissolved in DI water (6ml) and 1.02ml of formaldehyde solution (40%) was added to the base and stirred. This solution was then added to the slurry. This was then heated up to 90°C. Upon reaching this temperature, the flask was raised from the heating mantle. When the temperature had fallen to below 60°C, the stirring was also stopped. Upon reaching room temperature, the slurry was decanted. The flask was then refilled with DI water, stirred rapidly for 5 minutes and allowed to settle. The solution was then filtered and the resulting black solid was washed with 500ml of DI water.

The black solid was collected and dried in the oven at 90°C for 24 hours.

The diffraction pattern (not shown) indicates the presence of a minor amount of poorly crystalline Palladium (Pd, PDF No.00-046-1043).

### Example 13

### 0.5% Pd-Gly / Graphite

9.95g of Timcal T44 graphite was placed in a round bottomed flask and 60ml of water added to this. This was stirred for one hour before 0.146g of Na₂PdCl₄ (in 10 ml of DI water) was added to this. Glycine (0.035g) was then dissolved in 10ml of water and rapidly added to the slurry, which was stirred for a further 30 minutes. NaOH (0.062g) and formaldehyde solution (0.204ml) were combined and subsequently added to the slurry, before it was heated to 90°C. Upon reaching this temperature, excess water was added to bring the temperature down to 60°C. The resulting black solid was then filtered off, washed with water (500ml) and dried in the oven at 105°C for 2 days.

### ICP Analysis: Pd 0.42%

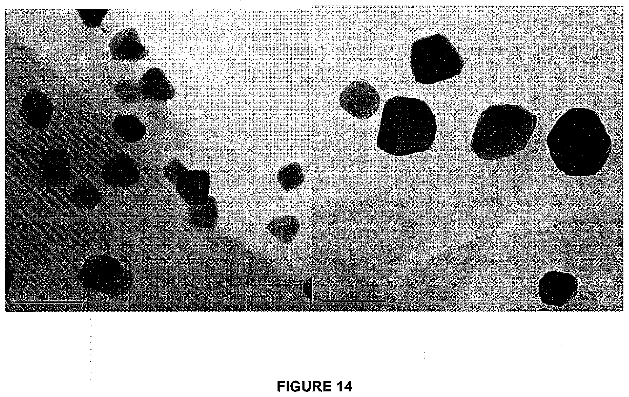

Figures 14a and 14b are TEM analyses of the glycine-modified Pd on graphite catalyst. The graphite supported catalysts have very large and faceted particles present, with an average particle size of approximately 40nm. A number of particles display clear grain boundaries, which without being bound by theory, may indicate that the particles grew together from two different nucleation points. In addition, several of the particles display a strange diffraction effect in the electron beam, which may be a result of their relatively large size.

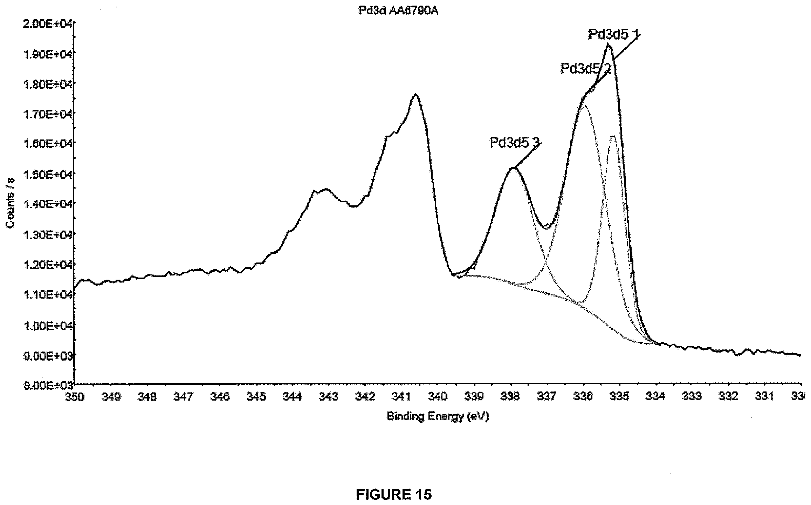

Figure 15 illustrates the XPS analysis of the glycine-modified Pd on graphite catalyst.

The above method was also used to prepare 1%, 2.5% and 5% loaded catalysts.

### Example 14

### Au-Pd-Lys / GSX (1:1 wt% with respect to each metal)

9.5g of Carbon GSX was suspended in 60ml of DI water. This was allowed to stand for one hour before stirring was commenced. The metal salts (Na₂PdCl₄ 0.73g and HAuCl₄ 0.62g) were dissolved in 10ml of water and rapidly added to the slurry. This was stirred for 30 minutes. Lysine monohydrate (0.61 g) was then dissolved in 10ml of water and rapidly added to the slurry and this was stirred for a further 30 minutes. NaOH (0.34g) and formaldehyde (0.85ml) were dissolved in 10 ml of water and this solution was added to the slurry, which was then heated to boiling. This was maintained at this temperature for 30 minutes. After this water was added to the slurry to bring the temperature down to 60°C. This was then allowed to cool to room temperature. The slurry was then filtered and washed with 500ml of water. The resulting black solid was collected and dried in the oven for 3 days.

### ICP Analysis: Au 2.60%, Pd 2.55%

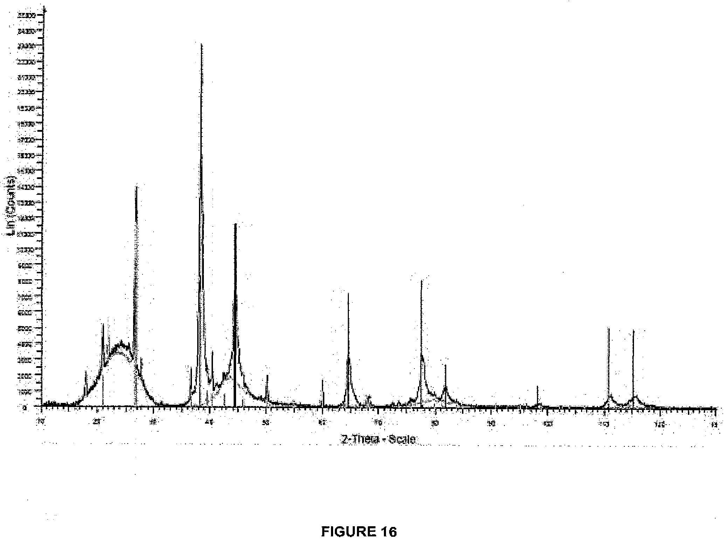

XRD: The diffraction pattern Figure 16 indicates the presence of a major amount of poorly crystalline Gold (Au, PDF No.00-004-0784) supported on GSX carbon. The Au crystallite size has been estimated by Rietveld analysis (LVol-IB method) to be ~9.9nm. There is no evidence of any crystalline Pd species.

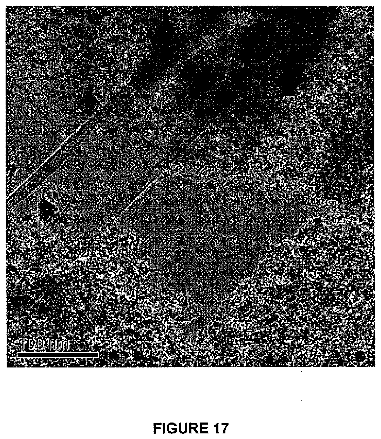

Figure 17 is the TEM analysis of the Au-Pd-Lys / GSX catalyst.

The above method has also been used to prepare Au-Pd-Lys / GSX catalysts with different metal ratios e.g. 0.5:1, 2:1, 4:1 and 9:1 wt% with respect to Au and Pd respectively, as well as different combinations of metals e.g. AuPt and PdPt.

### Example 15

### Catalysis Data

### a. N-debenzylation reactions

One aspect of the catalytic work has focussed on N-debenzylation reactions containing aryl chloride groups and chloronitrobenzene hydrogenation. In both cases, the conversion to chloroaniline proceeded with significantly less reaction of the aryl chloride functionality than standard catalysts. This was further benefited by the addition of hydrochloric acid to the reaction mixture, which further retarded the undesired dehalogenation.

The hydrogenation of N-benzyl protected 2-chloroaniline was studied. A range of conditions were examined, but the conditions of a 0.5M ethanol solution, reacting at 50°C, 1 bar H₂, with a 1:250 catalyst to substrate ratio were found to be suitable. The solutions contained 1,4-dioxane as an internal standard for the resulting analysis.

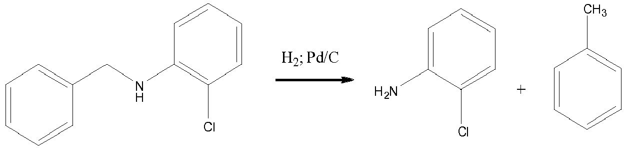

The reactions were performed in a Baskerville 10 Vessel Multicell Reactor using 5ml of the ethanolic solution. Where the addition of acid was required, 0.5ml of hydrochloric acid (1 molar equivalent wrt to substrate) was added to the reaction mixture. Analysis of the solutions was made using GCMS. The presence of acid often resulted on the precipitation of salts from the solution. In order to ensure all of the products could be analysed a basic extraction was performed prior to analysis. This involved the addition of 10M NaOH solution (~5ml) and dichloromethane (~10ml). Vigorous stirring and extraction of the organic layer allowed analysis that gave satisfactory mass balances to be obtained.

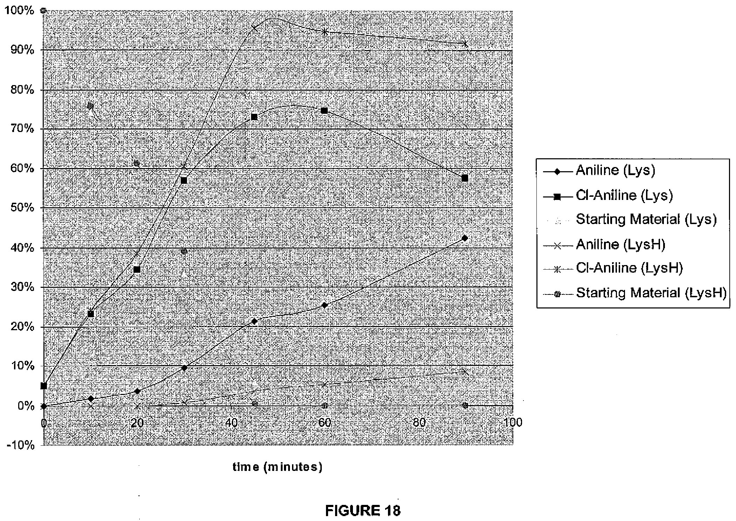

Analysis of the lysine-precipitated Pd/C catalyst was examined over different time periods in the N-debenzylation both in the presence (LysH) and absence (Lys) of one equivalent of HCl (see Figure 18).

Figure 18 shows that in the absence of acid the dehalogenation process is initially slowed; however, over time aniline begins to form, illustrating the slow cleavage of the aryl chloride bond. In contrast, in the presence of the acid the unwanted dehalogenation step is significantly retarded so that after 90 minutes there is over 90% of the desired 2-chloroaniline product.

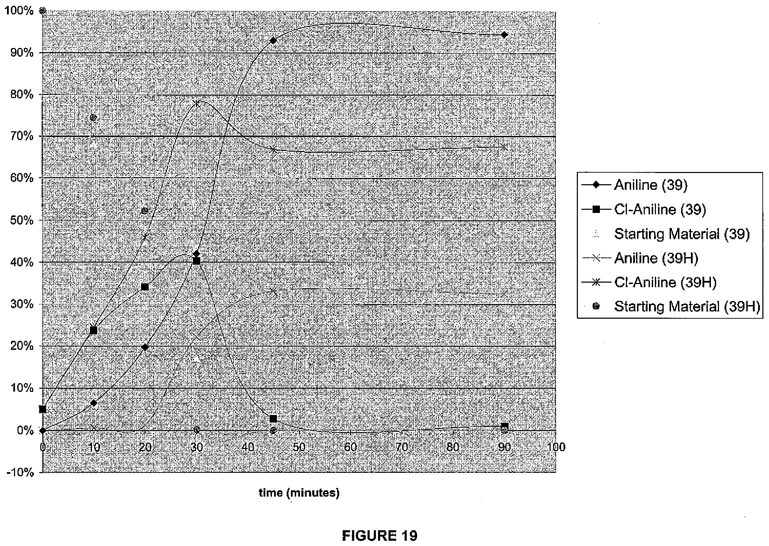

Analogous reactions were performed using a standard 5% Pd/C catalyst (Type 39), and these were again run in the presence (39H) and absence (39) of one molar equivalent of hydrochloric acid (Figure 19). In the absence of acid, dehalogenation was a rapid process with over 95% aniline formed after just 45 minutes. When acid was added, the dehalogenation process was slowed with 68% of the desired product at the end of the reaction, with the balance being aniline. However, this is dramatically less than in the amino acid precipitated catalyst.

Similar results were also shown to occur when using the analogous *meta* and *para* isomers.

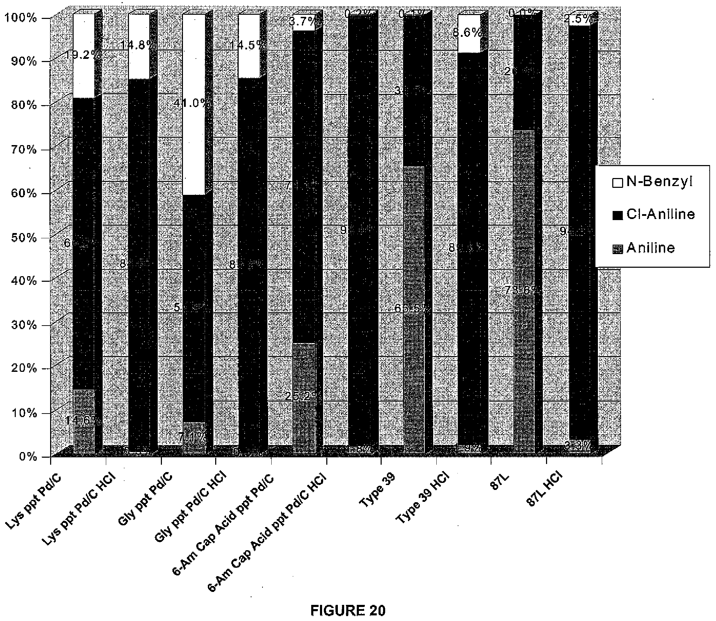

The reaction has also been examined using catalysts precipitated using different ligands. Figure 20 shows the same reaction conditions for the N-debenzylation run for one hour.

### b. Chloronitrobenzene Hydrogenation

The supported metal catalysts of the present invention have shown a propensity to retard dehalogenation reactions in chloronitrobenzene hydrogenations.

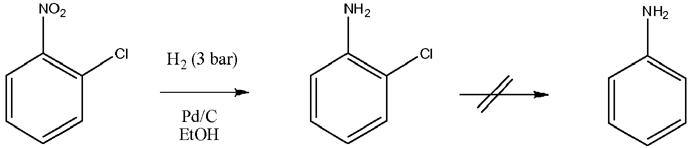

The reactions were performed in a Baskerville 10 Vessel Multicell Reactor using 5ml of the ethanolic solution. Where the addition of acid was required, 0.5ml of hydrochloric acid (1 molar equivalent wrt to substrate) was added to the reaction mixture. Analysis of the solutions was made using GC or GCMS. The presence of acid often resulted on the precipitation of salts from the solution. In order to ensure all of the products could be analysed a basic extraction was performed prior to analysis. This involved the addition of 10M NaOH solution (~5ml) and dichloromethane (~10ml). Vigorous stirring and extraction of the organic layer allowed analysis that gave satisfactory mass balances to be obtained.

The following data was taken after a 2.5 hour run, in order to ensure complete conversion of the starting materials.

| | **Aniline** | **2-Chloro-aniline** | **2-Chloro nitrobenzene** | **ClAn:An** |
|---|---|---|---|---|
| Pd-6-Amino Caproic Acid / C | 32.82% | 67.18% | 0.00% | 2.05 |
| Pd-1,6-Diaminohexane / C | 5.25% | 43.41% | 51.34% | 8.27 |
| Pd-Hexylamine / C | 42.75% | 57.25% | 0.00% | 1.34 |
| Pd-Lysine / C | 30.00% | 70.00% | 0.00% | 2.33 |
| Pd-Glycine / C | 22.82% | 77.18% | 0.00% | 3.38 |
| Pd-GlyGly / C | 19.80% | 72.60% | 0.00% | 3.67 |
| Pd-GlyGlyGly / C | 17.40% | 74.70% | 0.00% | 4.30 |
| Pt-Lysine / C | 9.40% | 79.60% | 0.00% | 8.48 |
| | | | | |
| Pt / C (Type 18) # (comparative) | 24.0% | 28.20% | 0.00% | 1.17 |
| Pd/C (Type 39) (comparative) | 88.84% | 11.16% | 0.00% | 0.13 |

| | | | | |
|---|---|---|---|---|
| 0.5M EtOH Solution, 50°C, 3 bar H₂ 1:1000 catalyst:substrate molar ratio Proportion of products via GCMS analysis ^{#}Note: Significant quantities of cyclohexylamine (8.3%) and dicyclohexylamine (27.8%) and other products (8.9%) were also observed in this transformation | | | | |

After this time, the standard Pd/C catalyst has resulted in near complete dehalogenation. In contrast, the amino modified catalysts all showed large quantities of the desired 2-chloroaniline - with the glycine modified catalysts appearing the best for this reaction.

Where the standard catalyst was used, the hydrogen uptake is rapid and overshoots the theoretical uptake required to just reduce the nitro group. The rate of reaction then slows, where it is believed that the aryl chloride groups are then reduced. This is confirmed by GCMS analysis after 2 hours which reveals that 80% of the reaction mixture is aniline.

In contrast, the hydrogen uptake of the lysine-modified catalyst slows earlier than the standard catalyst. The rate of reaction then also slows and it is believed that this is due to the slower dehalogenation occurring.

Addition of hydrochloric acid has also proved beneficial to this reaction, by minimising the rate of dehalogenation. The addition of one equivalent of hydrochloric acid to the reaction mixture resulted in an enhancement of the selectivity of the lysine-modified catalyst to over 90% selectivity.

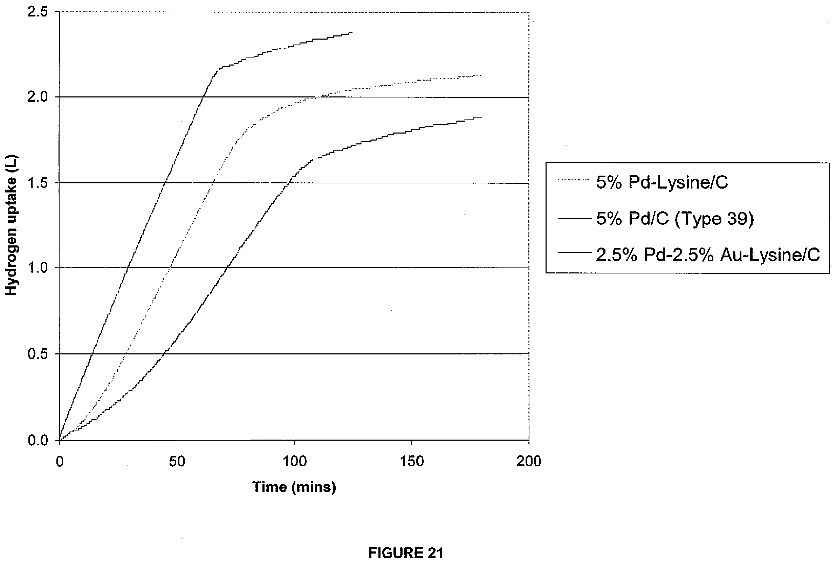

The use of bimetallic gold-palladium nanocatalysts has been shown to be beneficial to the palladium only system. The reduction in the undesirable dehalogenation of the substrate is observed in the hydrogen uptake curves (see Figure 21).

Reactions were undertaken using 60ml of a 0.5M ethanol solution of the substrate containing octane as an internal standard in a 100ml Parr autoclave. 60mg of the 5%, and 120mg of the 2.5% loaded Pd catalysts were used (1:1,000 catalyst to substrate molar ratio). Conditions used were a temperature of 50°C, a pressure of 3bar H₂ and mechanical stirring at 400rpm.

### c. Hydrogenation of Alkynes

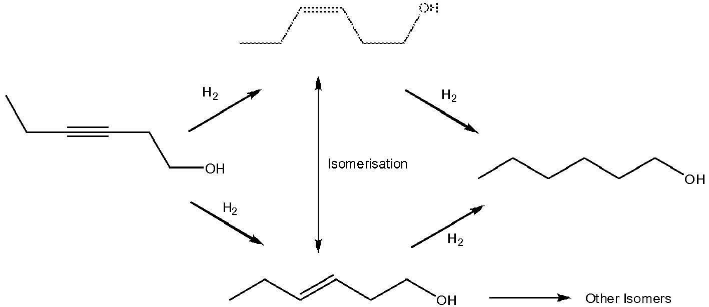

### Selective Hydrogenation of 3-Hexyn-1-ol

A number of palladium-based catalysts that were modified with amino acids were initially screened in a Baskerville 10 Vessel Multicell Reactor. This showed that a number of the amino acid modified catalysts gave high quantities of the desired cis-alkene product, in comparison with the standard lead-poisoned Lindlar catalyst. In contrast the analogous 5% Pd/C (87L) sample gave rise to near total over reduction to the unsaturated 1-hexanol.

**Table of Product Distribution, Mass Balance, Selectivity and Activity Data of 3-Hexyn-1-ol Hydrogenation using different amino acid modified catalysts. 0.5M EtOH solution, 30°C, 3 bar H₂, 30 mins, 1:1000 molar catalyst/substrate ratio, GC analysis**

| Modifier | trans-3-Hexen-1-ol | cis-3-Hexen-1-ol | 1-Hexanol | 3-Hexyn-1-ol | Mass Balance | Conversion | Selectivity |
|---|---|---|---|---|---|---|---|
| Glycine (10% Pd Loading) | 2.02% | 53.82% | 0.87% | 46.56% | 104.07% | 53.44% | 93.59% |
| Glycine (5% Pd Loading) | 3.09% | 83.85% | 1.13% | 13.58% | 102.79% | 86.42% | 94.00% |
| Glycine (1% Pd Loading) | 21.39% | 59.10% | 10.86% | 0.00% | 99.57% | 100.00% | 59.36% |
| Proline | 2.31% | 63.78% | 1.03% | 34.88% | 102.81% | 65.12% | 93.88% |
| Alanine | 6.68% | 89.08% | 2.60% | 0.00% | 101.26% | 100.00% | 87.98% |
| Arginine | 3.81% | 92.61% | 1.58% | 3.00% | 102.21% | 97.00% | 93.35% |
| Serine | 2.30% | 63.59% | 0.86% | 33.33% | 101.03% | 66.67% | 93.92% |
| D-Phenylalanine | 12.39% | 75.62% | 7.48% | 0.00% | 100.32% | 100.00% | 75.37% |
| L-Phenylalanine | 14.02% | 72.95% | 8.33% | 0.00% | 100.65% | 100.00% | 72.48% |
| 5% Pd/C (87L) * | 11.22% | 1.80% | 71.37% | 0.00% | 101.25% | 100.00% | 1.78% |
| Pd Glycine / Al₂O₃ | 2.13% | 74.12% | 0.87% | 25.94% | 103.65% | 74.06% | 95.37% |
| Pd Glycine/ Al₂O₃NaOH | 2.45% | 76.69% | 1.20% | 20.63% | 101.89% | 79.37% | 94.38% |
| Blank - no catalyst * | 0.00% | 0.14% | 0.07% | 97.71% | 97.92% | 2.29% | 68.13% |
| Pd Lys ppt | 1.53% | 40.59% | 0.63% | 61.73% | 104.98% | 38.27% | 93.84% |
| PdPb CaCO₃ (Lindlar's cat) * | 2.03% | 89.15% | 0.62% | 15.57% | 107.64% | 84.43% | 96.83% |

| | | | | | | | |
|---|---|---|---|---|---|---|---|
| **NB Support is Norit Carbon GSX and catalysts contain 5% metal by weight, unless stated otherwise.** *** Comparative** | | | | | | | |

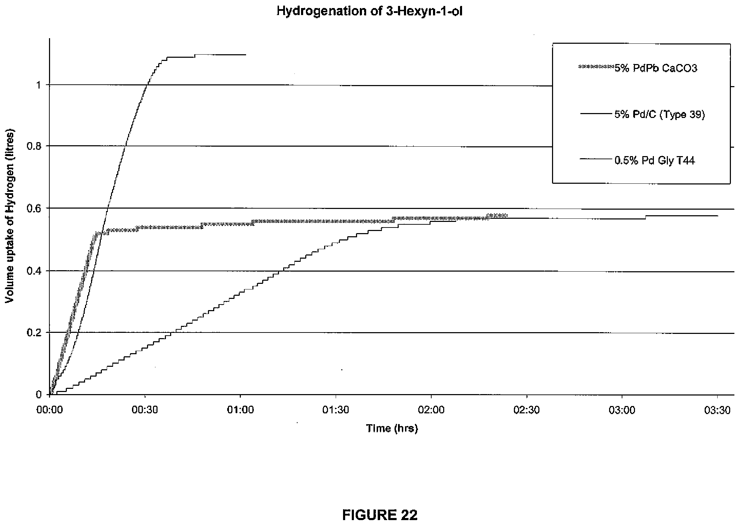

A number of samples were also probed in a Parr single autoclave, using the same reaction conditions. The hydrogen uptake curves for Lindlar catalyst (PdPb/CaCO₃), Pd/C (Type 39) and Pd-Gly / T44 are provided in Figure 22.

## Claims

1. A supported metal catalyst, wherein the catalyst comprises at least one metal selected from Group VIII or IB of the Periodic Table, the catalyst is modified by at least one amine, and wherein the catalyst comprises faceted particles,
provided that when the metal is Pt and the support is Al₂O₃, the amine is not:
a) S-benzyl-L-cysteine;
b) N-benzyl-S-benzyl-L-cysteine;
c) L-cysteine ethyl ester;
d) S-benzyl-L-cysteine ethyl ester;
e) N-benzyl-S-benzyl-L-cysteine ethyl ester;
f) S-phenyl-L-cysteine ethyl ester; or
g) N-benzyl-S-phenyl-L-cysteine ethyl ester.

2. A catalyst according to claim 1, wherein the support is selected from the group consisting of carbon, alumina, calcium carbonate, titania, silica, zirconia, ceria and a combination thereof.

3. A catalyst according to claim 1 or claim 2, wherein the metal is selected from the group consisting of ruthenium, rhodium, palladium, osmium, iridium, platinum, gold, silver, copper, iron, cobalt, nickel and a combination thereof.

4. A catalyst according to any one of the preceding claims, wherein the amine is selected from the group consisting of natural amino acids, non-natural amino acids, peptides, substituted or unsubstituted alkylamines, substituted or unsubstituted alkyldiamines, substituted or unsubstituted alkylpolyamines and combinations thereof.

5. A catalyst according to claim 4, wherein the amine is selected from the group consisting of lysine, glycine, proline, alanine, serine, phenylalanine, asparagine, aspartic acid, valine, butylamine, 6-aminocaproic acid, 1,6-diaminohexane, hexylamine and combinations thereof.

6. A catalyst according to any one of the preceding claims, wherein the catalyst is:
| **Metal(s)** | **Support** | **Amine** |
|---|---|---|
| Palladium | Carbon | Lysine |
| Palladium | Carbon | Glycine |
| Palladium | Carbon | Proline |
| Palladium | Carbon | Alanine |
| Palladium | Carbon | Arginine |
| Palladium | Carbon | Serine |
| Palladium | Carbon | Phenylalanine |
| Palladium | Carbon | Asparagine |
| Palladium | Carbon | Aspartic acid |
| Palladium | Carbon | Valine |
| Palladium | Carbon | Butylamine |
| Palladium | Carbon | 6-Amino caproic acid |
| Palladium | Carbon | 1,6-Diaminohexane |
| Palladium | Carbon | Hexylamine |
| Palladium | Alumina | Glycine |
| Palladium | Carbon | Gly-Gly |
| Palladium | Carbon | Gly-Gly-Gly |
| Platinum | Carbon | Lysine |
| Gold | Carbon | Lysine |
| Gold-palladium | Carbon | Lysine |
| Gold-platinum | Carbon | Lysine |
| Palladium-platinum | Carbon | Lysine |

7. A catalyst according to any one of the preceding claims, wherein the catalyst comprises crystallites.

8. A catalyst according to claim 7, wherein the size of the crystallites is from about 1 nm to about 50 nm.

9. A process for the preparation of a supported metal catalyst as defined in any one of claims 1 to 8, wherein the process comprises the steps of:
a) mixing a support, at least one water soluble metal salt and at least one amine in an aqueous solvent; and
b) adding a reducing agent to form the supported metal catalyst;
and wherein steps (a) and (b) are carried out as a one-pot reaction.

10. A process according to claim 9, wherein the at least one water soluble metal salt is selected from the group consisting of:
(i) M₂PtX₂ wherein M is H, Li, Na, K or NH₃ and X is Cl, Br, I, NO₃, OH or CN;
(ii) M₂PtX₆ wherein M is H, Li, Na, K or NH₃ and X is Cl, Br, I, NO₃, OH or CN;
(iii) PtX₂ wherein X is Cl, Br, I, NO₃, OH or CN;
(iv) PtX₄ wherein X is Cl, Br, I, NO₃, OH or CN;
(v) Pt(NH₃)_{4-y}X_{y} wherein X is Cl, Br, I or NO₃ and y is 0, 1, 2, 3 or 4;
(vi) M₂PdX₄ wherein M is H, Li, Na, K or NH₃ and X is Cl, Br, I, NO₃, OH, CN or HCO₃;
(vii) M₂PdX₆ wherein M is H, Li, Na, K or NH₃ and X is Cl, Br, I, NO₃, OH or CN;
(viii) PdX₂ wherein X is Cl, Br, I, NO₃, OH or CN;
(ix) MAuX₄ wherein M is H, Li, Na or K and X is Cl, Br or I;
(x) AuX₃ wherein X is OAc, Cl, Br, I or OH;
(xi) AuX wherein X is Cl, Br, I or CN;
(xii) RhX₃ wherein X is Cl, Br, I or NO₃;
(xiii) RuX₃ wherein X is Cl, Br or I;
(xiv) NiX₂ wherein X is F, Cl, Br, I, OH, OAc or NO₃; and
(xv) Pd(oxalate), Ni(oxalate), Ni(oxalate).2H₂O, [Rh(OAc)₂]₂, NiCO₃, Ni (citrate).xH₂O.

11. A process according to claim 9 or claim 10, wherein the reducing agent is (i) a combination of a base and formaldehyde, (ii) a formate, (iii) a borohydride, (iv) a hypophosphite, (v) hydrazine, or (vi) hydrogen.

12. A process according to any one of claims 9 to 11, wherein step (a) and step (b) are carried out at one or more temperatures between about 15°C and 100°C.

13. A process for the preparation of an optionally substituted amine, comprising the step of hydrogenating an optionally substituted benzyl-amine in the presence of hydrogen and a supported metal catalyst as claimed in any one of claims 1 to 8.

14. A process for the preparation of an optionally substituted arylamine, comprising the step of hydrogenating an optionally substituted aryl compound comprising one or more nitro groups in the presence of hydrogen and a supported metal catalyst as defined in any one of claims 1 to 8.

15. A process for the preparation of an optionally substituted alkene, comprising the step of hydrogenating an optionally substituted alkyne in the presence of hydrogen and a supported metal catalyst as defined in any one of claims 1 to 8.

## Patentansprüche

1. Geträgerter Metallkatalysator, wobei der Katalysator mindestens ein Metall umfasst, das aus der Gruppe VIII oder IB des Periodensystems ausgewählt ist, wobei der Katalysator mit mindestens einem Amin modifiziert ist, und wobei der Katalysator facettierte Partikel umfasst,
mit der Maßgabe, dass das Amin, wenn das Metall Pt ist und der Träger Al₂O₃ ist, nicht den folgenden entspricht:
a) S-Benzyl-L-cystein;
b) N-Benzyl-S-benzyl-L-cystein;
c) L-Cystein-Ethylester;
d) S-Benzyl-L-cystein-Ethylester;
e) N-Benzyl-S-benzyl-L-cystein-Ethylester;
f) S-Phenyl-L-cystein-Ethylester; oder
g) N-Benzyl-S-phenyl-L-cystein-Ethylester.

2. Katalysator nach Anspruch 1, wobei der Träger aus der Gruppe ausgewählt ist, die aus Kohlenstoff, Aluminiumoxid, Calciumcarbonat, Titandioxid, Siliciumdioxid, Zirkoniumdioxid, Cerdioxid und einer Kombination davon besteht.

3. Katalysator nach Anspruch 1 oder Anspruch 2, wobei das Metall aus der Gruppe ausgewählt ist, die aus Ruthenium, Rhodium, Palladium, Osmium, Iridium, Platin, Gold, Silber, Kupfer, Eisen, Kobalt, Nickel und einer Kombination davon besteht.

4. Katalysator nach einem beliebigen der vorhergehenden Ansprüche, wobei das Amin aus der Gruppe ausgewählt ist, die aus den natürlichen Aminosäuren, nicht-natürlichen Aminosäuren, Peptiden, substituierten oder nicht substituierten Alkylaminen, substituierten oder nicht substituierten Alkyldiaminen, substituierten oder nicht substituierten Alkylpolyaminen und Kombinationen davon besteht.

5. Katalysator nach Anspruch 4, wobei das Amin aus der Gruppe ausgewählt ist, die aus Lysin, Glycin, Prolin, Alanin, Serin, Phenylalanin, Asparagin, Asparaginsäure, Valin, Butylamin, 6-Aminocapronsäure, 1,6-Diaminohexan, Hexylamin und Kombinationen davon besteht.

6. Katalysator nach einem beliebigen der vorhergehenden Ansprüche, wobei der Katalysator folgendermaßen beschaffen ist:
| **Metal(le)** | **Träger** | **Amine** |
|---|---|---|
| Palladium | Kohlenstoff | Lysin |
| Palladium | Kohlenstoff | Glycin |
| Palladium | Kohlenstoff | Prolin |
| Palladium | Kohlenstoff | Alanin |
| Palladium | Kohlenstoff | Arginin |
| Palladium | Kohlenstoff | Serin |
| Palladium | Kohlenstoff | Phenylalanin |
| Palladium | Kohlenstoff | Asparagin |
| Palladium | Kohlenstoff | Asparaginsäure |
| Palladium | Kohlenstoff | Valin |
| Palladium | Kohlenstoff | Butylamin |
| Palladium | Kohlenstoff | 6-Aminocapronsäure |
| Palladium | Kohlenstoff | 1,6-Diaminohexan |
| Palladium | Kohlenstoff | Hexylamin |
| Palladium | Aluminiumoxid | Glycin |
| Palladium | Kohlenstoff | Gly-Gly |
| Palladium | Kohlenstoff | Gly-Gly-Gly |
| Platin | Kohlenstoff | Lysin |
| Gold | Kohlenstoff | Lysin |
| Gold-Palladium | Kohlenstoff | Lysin |
| Gold-Platin | Kohlenstoff | Lysin |
| Palladium-Platin | Kohlenstoff | Lysin |

7. Katalysator nach einem beliebigen der vorhergehenden Ansprüche, wobei der Katalysator Kristallite umfasst.

8. Katalysator nach Anspruch 7, wobei die Größe der Kristallite ungefähr 1 nm bis ungefähr 50 nm beträgt.

9. Verfahren zur Herstellung eines geträgerten Metallkatalysators gemäß der Begriffsbestimmung in einem beliebigen der Ansprüche 1 bis 8, wobei das Verfahren die folgenden Schritte umfasst:
a) Vermischen eines Trägers, mindestens eines wasserlöslichen Metallsalzes und mindestens eines Amins in einem wässrigen Lösungsmittel; und
b) Hinzufügen eines Reduktionsmittels, um den geträgerten Metallkatalysator zu bilden;
und wobei die Schritte (a) und (b) als Eintopfreaktion durchgeführt werden.

10. Verfahren nach Anspruch 9, wobei das mindestens eine wasserlösliche Metallsalz aus der Gruppe ausgewählt ist, die aus den folgenden besteht:
(i) M₂PtX₂,wobei M gleich H, Li, Na, K oder NH₃ ist und X gleich Cl, Br, I, NO₃, OH oder CN ist;
(ii) M₂PtX₆,wobei M gleich H, Li, Na, K oder NH₃ ist und X gleich Cl, Br, I, NO₃, OH oder CN ist;
(iii) PtX₂, wobei X gleich Cl, Br, I, NO₃, OH oder CN ist;
(iv) PtX₄, wobei X gleich Cl, Br, I, NO₃, OH oder CN ist;
(v) Pt(NH₃)₄-yXy,wobei X gleich Cl, Br, I oder NO₃ ist und y gleich 0, 1, 2, 3 oder 4 ist;
(vi) M₂PdX₄, wobei M gleich H, Li, Na, K oder NH₃ ist und X gleich Cl, Br, I, NO₃, OH, CN oder HCO₃ ist;
(vii) M₂PdX₆, wobei M gleich H, Li, Na, K oder NH₃ ist und X gleich Cl, Br, I, NO₃, OH oder CN ist;
(viii) PdX₂, wobei X gleich Cl, Br, I, NO₃, OH oder CN ist;
(ix) MAuX₄ wobei M gleich H, Li, Na oder K ist und X gleich Cl, Br oder I ist;
(x) AuX₃, wobei X gleich OAc, Cl, Br, I oder OH ist;
(xi) AuX, wobei X gleich Cl, Br, I oder CN ist;
(xii) RhX₃, wobei X gleich Cl, Br, I oder NO₃ ist;
(xiii) RuX₃, wobei X gleich Cl, Br oder I ist;
(xiv) NiX₂, wobei X gleich F, Cl, Br, I, OH, OAc oder NO₃ ist; und
(xv) Pd(oxalat), Ni(oxalat), Ni(oxalat) · 2H₂O, [Rh(OAc)₂]₂, NiCO₃, Ni(citrat) · xH₂O.

11. Verfahren nach Anspruch 9 oder Anspruch 10, wobei es sich bei dem Reduktionsmittel um (i) eine Kombination aus einer Basis und Formaldehyd, (ii) ein Formiat, (iii) ein Borhydrid, (iv) ein Hypophosphat, (v) Hydrazin oder (vi) um Wasserstoff handelt.

12. Verfahren nach einem beliebigen der Ansprüche 9 bis 11, wobei der Schritt (a) und der Schritt (b) bei einer oder mehreren Temperaturen zwischen ungefähr 15 °C und 100 °C durchgeführt werden.

13. Verfahren zur Herstellung eines möglicherweise substituierten Amins, wobei es den Schritt des Hydrierens eines möglicherweise substituierten Benzylamins in Gegenwart von Wasserstoff und eines geträgerten Metallkatalysators umfasst, wie er in einem beliebigen der Ansprüche 1 bis 8 beansprucht ist.

14. Verfahren zur Herstellung eines möglicherweise substituierten Arylamins, wobei es den Schritt des Hydrieren einer möglicherweise substituierten Arylverbindung, die eine oder mehrere Nitrogruppen umfasst, in Gegenwart von Wasserstoff und eines geträgerten Metallkatalysators gemäß der Begriffsbestimmung in einem beliebigen der Ansprüche 1 bis 8 umfasst.

15. Verfahren zur Herstellung eines möglicherweise substituierten Alkens, wobei es den Schritt des Hydrieren eines möglicherweise substituierten Alkins in Gegenwart von Wasserstoff und eines geträgerten Metallkatalysators gemäß der Begriffsbestimmung in einem beliebigen der Ansprüche 1 bis 8 umfasst.

## Revendications

1. Catalyseur supporté à base de métaux, le catalyseur comprenant au moins un métal choisi dans le Groupe VIII ou IB du Tableau Périodique, le catalyseur étant modifié par au moins une amine, et le catalyseur comprenant des particules facetées,
à condition que lorsque le métal est Pt et le support est Al₂O₃, l'amine ne soit pas :
a) de la S-benzyl-L-cystéine ;
b) de la N-benzyl-S-benzyl-L-cystéine ;
c) de l'ester éthylique de L-cystéine ;
d) de l'ester éthylique de S-benzyl-L-cystéine ;
e) de l'ester éthylique de N-benzyl-S-benzyl-L-cystéine ;
f) de l'ester éthylique de S-phényl-L-cystéine ; ou
g) de l'ester éthylique de N-benzyl-S-phényl-L-cystéine.

2. Catalyseur selon la revendication 1, dans lequel le support est choisi dans le groupe constitué par le carbone, l'alumine, le carbonate de calcium, le dioxyde de titane, la silice, la zircone, le dioxyde de cérium et une combinaison de ceux-ci.

3. Catalyseur selon la revendication 1 ou la revendication 2, dans lequel le métal est choisi dans le groupe constitué par le ruthénium, le rhodium, le palladium, l'osmium, l'iridium, le platine, l'or, l'argent, le cuivre, le fer, le cobalt, le nickel et une combinaison de ceux-ci.

4. Catalyseur selon l'une quelconque des revendications précédentes, dans lequel l'amine est choisie dans le groupe constitué par les acides aminés naturels, les acides aminés non naturels, les peptides, les alkylamines substituées ou non substituées, les alkyldiamines substituées ou non substituées, les alkylpolyamines substituées ou non substituées et les combinaisons de ceux-ci.

5. Catalyseur selon la revendication 4, dans lequel l'amine est choisie dans le groupe constitué par la lysine, la glycine, la proline, l'alanine, la sérine, la phénylalanine, l'asparagine, l'acide aspartique, la valine, la butylamine, l'acide 6-aminocaproïque, le 1,6-diaminohexane, l'hexylamine et les combinaisons de ceux-ci.

6. Catalyseur selon l'une quelconque des revendications précédentes, le catalyseur étant :
| **Métal (Métaux)** | **Support** | **Amine** |
|---|---|---|
| Palladium | Carbone | Lysine |
| Palladium | Carbone | Glycine |
| Palladium | Carbone | Proline |
| Palladium | Carbone | Alanine |
| Palladium | Carbone | Arginine |
| Palladium | Carbone | Sérine |
| Palladium | Carbone | Phénylalanine |
| Palladium | Carbone | Asparagine |
| Palladium | Carbone | Acide aspartique |
| Palladium | Carbone | Valine |
| Palladium | Carbone | Butylamine |
| Palladium | Carbone | Acide 6-aminocaproïque |
| Palladium | Carbone | 1,6-diaminohexane |
| Palladium | Carbone | Hexylamine |
| Palladium | Alumine | Glycine |
| Palladium | Carbone | Gly-Gly |
| Palladium | Carbone | Gly-Gly-Gly |
| Platine | Carbone | Lysine |
| Or | Carbone | Lysine |
| Or-Palladium | Carbone | Lysine |
| Or-Platine | Carbone | Lysine |
| Palladium-platine | Carbone | Lysine |

7. Catalyseur selon l'une quelconque des revendications précédentes, le catalyseur comprenant des cristallites.

8. Catalyseur selon la revendication 7, dans lequel la taille des cristallites est d'environ 1 nm à environ 50 nm.

9. Procédé de préparation d'un catalyseur supporté à base de métaux tel que défini dans l'une quelconque des revendications 1 à 8, le procédé comprenant les étapes de :
a) mélanger un support, au moins un sel métallique hydrosoluble et au moins une amine dans un solvant aqueux ; et
b) ajouter un réducteur pour former le catalyseur supporté à base de métaux ;
et les étapes (a) et (b) étant réalisées sous forme de réaction en récipient unique.

10. Procédé selon la revendication 9, dans lequel l'au moins un sel métallique hydrosoluble est choisi dans le groupe constitué par :
(i) M₂PtX₂ dans lequel M est H, Li, Na, K ou NH₃ et X est Cl, Br, I, NO₃, OH ou CN ;
(ii) M₂PtX₆ dans lequel M est H, Li, Na, K ou NH₃ et X est Cl, Br, I, NO₃, OH ou CN ;
(iii) PtX₂ dans lequel X est Cl, Br, I, NO₃, OH ou CN ;
(iv) PtX₄ dans lequel X est Cl, Br, I, NO₃, OH ou CN ;
(v) Pt(NH₃)_{4-y}X_{y} dans lequel X est Cl, Br, I ou NO₃ et y est 0, 1, 2, 3 ou 4 ;
(vi) M₂PdX₄ dans lequel M est H, Li, Na, K ou NH₃ et X est Cl, Br, I, NO₃, OH, CN ou HCO₃ ;
(vii) M₂PdX₆ dans lequel M est H, Li, Na, K ou NH₃ et X est Cl, Br, I, NO₃, OH ou CN ;
(viii) PdX₂ dans lequel X est Cl, Br, I, NO₃, OH ou CN ;
(ix) MAuX₄ dans lequel M est H, Li, Na ou K et X est Cl, Br ou I ;
(x) AuX₃ dans lequel X est OAc, CI, Br, I ou OH ;
(xi) AuX dans lequel X est CI, Br, I ou CN ;
(xii) RhX₃ dans lequel X est Cl, Br, I ou NO₃ ;
(xiii) RuX₃ dans lequel X est Cl, Br ou I ;
(xiv) NiX₂ dans lequel X est F, CI, Br, I, OH, OAc ou NO₃; et
(xv) Pd(oxalate), Ni(oxalate), Ni(oxalate).2H₂O, [Rh(OAc)₂]₂, NiCO₃, Ni (citrate).xH₂O.

11. Procédé selon la revendication 9 ou la revendication 10, dans lequel le réducteur est (i) une combinaison d'une base et de formaldéhyde, (ii) un formiate, (iii) un borohydrure, (iv) un hypophosphite, (v) de l'hydrazine, ou (vi) de l'hydrogène.

12. Procédé selon l'une quelconque des revendications 9 à 11, dans lequel l'étape (a) et l'étape (b) sont effectuées à une ou plusieurs températures entre environ 15 °C et 100 °C.

13. Procédé de préparation d'une amine éventuellement substituée, comprenant l'étape d'hydrogénation d'une benzylamine éventuellement substituée en présence d'hydrogène et d'un catalyseur supporté à base de métaux tel que revendiqué dans l'une quelconque des revendications 1 à 8.

14. Procédé de préparation d'une arylamine, éventuellement substituée, comprenant l'étape d'hydrogénation d'un composé arylique éventuellement substitué comprenant un ou plusieurs groupes nitro en présence d'hydrogène et d'un catalyseur supporté à base de métaux tel que défini dans l'une quelconque des revendications 1 à 8.

15. Procédé de préparation d'un alcène éventuellement substitué, comprenant l'étape d'hydrogénation d'un alcyne éventuellement substitué en présence d'hydrogène et d'un catalyseur supporté à base de métaux tel que défini dans l'une quelconque des revendications 1 à 8.
